# EUROPÄISCHE PATENTSCHRIFT

(11) **EP 4 543 361 B1**
(45) Veröffentlichungstag und Bekanntmachung des Hinweises auf die Patenterteilung: **05.08.2026**
(21) Anmeldenummer: 23736635.6
(22) Anmeldetag: 27.06.2023
(51) Int. Cl.: A61C 17/22, A61C 17/02, A61C 19/06, A61C 7/00

(54) **ULTRASCHALLSYSTEM ZUM REINIGEN UND/ODER REGENERIEREN VON EIN ODER MEHREREN STRUKTUREN INNERHALB EINER MUNDHÖHLE**
ULTRASOUND SYSTEM FOR CLEANING AND/OR REGENERATING ONE OR MORE STRUCTURES INSIDE AN ORAL CAVITY
SYSTÈME À ULTRASONS DE NETTOYAGE ET/OU DE RÉGÉNÉRATION D'UNE OU DE PLUSIEURS STRUCTURES À L'INTÉRIEUR D'UNE CAVITÉ BUCCALE

(30) Priorität: 27.06.2022 DE 102022115920
(43) Veröffentlichungstag der Anmeldung: 30.04.2025
(73) Patentinhaber: Fraunhofer-Gesellschaft zur Förderung der angewandten Forschung e.V., 80686 München (DE)
(72) Erfinder: BEN ACHOUR, Anas, 01257 Dresden (DE); SCHRÖDER, Tom Alexander, 01307 Dresden (DE); LAUER, Günter, 01326 Dresden (DE)
(74) Vertreter: Viering, Jentschura & Partner mbB Patent- und Rechtsanwälte
(86) Internationale Anmeldenummer: PCT/EP2023/067488
(87) Internationale Veröffentlichungsnummer: WO 2024/003056

(56) Entgegenhaltungen:
- WO-A1-2007/121760
- US-A1- 2008 233 541
- US-A1- 2020 121 420
- US-A1- 2021 369 431

## Beschreibung

Verschiedene Ausführungsformen betreffen ein Ultraschallsystem und Verfahren zum Reinigen und/oder Regenerieren von ein oder mehreren Strukturen innerhalb einer Mundhöhle. Die Erfindung betrifft jedoch nur das Ultraschallsystem.

Im Allgemeinen kann eine unzureichende Zahnhygiene nicht nur einen bedeutenden Einfluss auf die Zahngesundheit, sondern auch auf den allgemeinen Gesundheitszustand eines Menschen haben. Eine ausreichende Reinigung der Zähne ist in hohem Maß von der Motivation, Kontinuität und der korrekten Anwendung der Zahnbürsten und der Interdentalraumpflege abhängig. Es kann durchaus Personen geben, welche die genannten Anforderungen nicht erfüllen können, und daher bei der täglichen Zahnreinigung auf die Hilfe anderer angewiesen sind.

Trotz des technischen Fortschritts basiert die tägliche Zahnreinigung zum überwiegenden Anteil auf dem Prinzip des mechanischen Abriebs. Dies wird in aller Regel von manuell geführten Handzahnbürsten in Kombination mit einer Zahnpasta durchgeführt. Die Inhaltsstoffe der Zahnpasta sind üblicherweise auf eine Remineralisierung des Zahnschmelzes, Entzündungshemmung und Erhöhung der Reinigungsleistung (z.B. mittels sogenannter Putzkörperchen) gerichtet. Bei der Zahnreinigung können die Technik des Ausführenden, die Zeitdauer und Anzahl der Putzvorgänge einen bedeutenden Einfluss auf das Reinigungsergebnis haben. So können beispielsweise unzureichende Entfernungen von Verunreinigungen (wie beispielsweise eines Biofilms, oder eines bereits mineralisierten Biofilms (Zahnstein)) oder Schädigungen des Zahnschmelzes durch zu hohe Abrasionskräfte beim Putzen in Anwesenheit von Putzkörpern der Zahnpasta entstehen. Beispielsweise können technisch unterstützte Handzahnbürsten diese nachteiligen Effekte durch eine direkte Rückmeldung an den Anwender bereits zum Teil ausgleichen (z.B. durch integrierte Zeitmesser und/oder Kraftmesser).

Kommerziell verfügbare (Ultra-)Schallzahnbürsten basieren im Allgemeinen auf dem Prinzip der Schwingung eines Bürstenkopfes bzw. einer bestimmten geometrischen Anordnung von Borsten. Diese können durch Schwingungen des Bürstenkopfes eine Relativbewegung zwischen den Borsten und den Zähnen erzeugen, wodurch ebenso wie bei der manuellen Putztechnik Zahnbelag durch Abrasion entfernt und die Zähne gereinigt werden können. Neuere Ultraschallzahnbürsten können in einem wesentlich höheren Frequenzbereich schwingen, wodurch im Wesentlichen eine Relativbewegung der Borsten (anstelle des gesamten Bürstenkopfes) stattfindet. Die Reinigung der Zahnoberflächen und des Zahnfleischs kann hierbei durch Kavitationsbläschen im Speichel bzw. der Zahnpasta, welche in Folge der Schallwellen implodieren, erzeugt werden.

Als ein Nachteil derartiger Zahnbürsten kann beispielsweise die Bauform betrachtet werden, die im Wesentlichen der einer klassischen Zahnbürste entspricht und somit das Putzergebnis stark von der konsequenten und richtigen Benutzung des Anwenders abhängig ist. Im Detail wird ein konsequentes Abfahren jedes Zahnes benötigt und durch die manuelle Führung der Bürste kann möglicherweise kein optimales Schallwellenfeld in dem zu reinigenden Bereich erreicht werden. Infolgedessen kann die Reinigung unzureichend sein. Weiterhin besteht die Gefahr des zu starken oder zu schwachen Andrückens der Zahnbürste an die Zähne, sowie einer ungenügenden zeitlichen Einteilung des Putzvorgangs. So können beispielsweise bestimmte Bereiche zu lang und andere Bereiche zu kurz und somit unzureichend geputzt werden. Zudem weisen die herkömmlichen (Ultraschallzahnbürsten) üblicherweise keine zielgerichtete Einstellbarkeit einer Schwingungsfrequenz im ultrafrequenten Bereich auf, um das akustische Feld auf die Bedürfnisse eines Nutzers gezielt anzupassen. Ein weiterer maßgeblicher Nachteil der klassischen Zahnbürsten-Bauform ist eine üblicherweise ungenügende Reinigung der Zwischenräume der Zähne. Diese kann im Allgemeinen lediglich über die zusätzliche Anwendung von interdentalraumbürsten und/oder Zahnseide zufriedenstellend erreicht werden.

Ein Ziel des Zähneputzens ist unter anderem, eine Bildung eines Biofilms und/oder von Zahnstein auf den Zähnen durch eine regelmäßige Entfernung zu verhindern. Zahnstein kann beispielsweise in mehreren Schritten entstehen: 1. Proteinadsorption, 2. Bakterienadhäsion, 3. Bakterielle Biofilmbildung und Reifung, und 4. Mineralisierung des gereiften Biofilms (z.B. sogenannten Plaques), wodurch der eigentliche Zahnstein entsteht. Anschaulich bedeutet das, dass aufgrund einer ausbleibenden Reinigung die bakteriellen Stoffwechselprodukte (v.a. Säuren) zu einer Schädigung der Zahnsubstanz und somit in ultima ratio zum vollständigen Zahnverlust führen können. Ein Verlust von Zähnen führt im Allgemeinen direkt zu einer Minderung der Lebensqualität des Betroffenen und verursacht im Falle eines Zahnersatzes erhebliche zusätzliche Kosten.

US 2020/0121420 A1 beschreibt Ultraschallgeräte, -systeme und -verfahren für die kieferorthopädische Behandlung, die mit Alignern durchgeführt wird. Die Kommunikation der gewünschten Ultraschallbehandlung kann von einem Aligner zu einem Ultraschallgerät durch ein Kommunikationsmodul unter Verwendung einer Identifikationsnummer auf dem Aligner erfolgen. Ferner ist eine Zahnspange mit Ultraschall-Wandlern offenbart.

WO 2007/121760 A1 beschreibt eine zahnärztliche Vorrichtung, die eine Schale umfasst, die zum Empfangen von Zähnen und/oder Zahnfleisch eines oder beider Zahnbögen geformt sind, wobei die Schale mit einem oder mehreren Vorsprüngen versehen ist, die so konfiguriert sind, dass sie die Zähne und/oder das Zahnfleisch physikalisch stimulieren, und ein Gehäuse, das so geformt ist, dass es die Schale aufnimmt, wobei das Gehäuse mit einem oder mehreren Wandlern versehen ist, die so konfiguriert sind, dass sie die Vorsprünge mechanisch stimulieren, und optional mit Mitteln zum Bereitstellen von Licht versehen ist.

US 2021/0369431 A1 offenbart eine Mundstückreinigungsvorrichtung, die ein U-förmiges Mundstück aufweist, das gegenüberliegende Kanäle zum Aufnehmen der Zähne eines Benutzers darin aufweist. Untraschallwandler und Ultraviolett(UV)-Leuchtdioden sind an einer den Zähnen zugewandten Oberfläche der Kanäle angeordnet. Ferner ist eine Steuervorrichtung vorgesehen zum Ansteuern der Untraschallwandler, um ein vordefiniertes Ultraschallsignal gemäß einem vorbestimmten Betriebsmodus auszuwählen, das an die Zähne angelegt werden kann.

Ferner beschreibt US 2008/0233541 A1 eine autonome Vorrichtung, die eine Schale umfasst, die geformt ist für das Aufnehmen von Zähnen und/oder Zahnfleisch eines Zahnbogens. Die Vorrichtung ist nützlich für Verfahren zum Bleichen der Zähne, zur Schmerzlinderung, zur Behandlung von Bakterien, und zur Behandlung von Mundgeruch und Heilung.

Der Gegenstand der Erfindung wird durch das Ultraschallsystem nach Anspruch 1 definiert.

Die Verfahren zum Reinigen und/oder zur Regeneration der verschiedenen Aspekten sind von der beanspruchten Erfindung ausgeschlossen.

Gemäß verschiedenen Aspekten werden eine Vorrichtung und ein Verfahren bereitgestellt, mittels derer die tägliche Reinigung von Zähnen (z.B. ein Entfernen eines Biofilms und/oder von Zahnstein von Glattflächen und/oder aus Zwischenräumen der Zähne) verbessert werden kann. Gemäß verschiedenen Aspekten wird eine Vorrichtung und ein Verfahren bereitgestellt, die umfassende Funktionalitäten zur Verbesserung einer Mundhygiene eines Anwenders bereitstellen.

Gemäß verschiedenen Aspekten wird eine Vorrichtung bereitgestellt, die mehrere vorbestimmte Programme zur Stimulation eines Regenerationsverhaltens von Zähnen, eines Zahnhalteapparats (z.B. des Zahnfleischs) und eines Kieferknochens ermöglicht.

Gemäß verschiedenen Aspekten werden eine Vorrichtung und ein Verfahren bereitgestellt, welche eine gezielte Anwendung von Ultraschallwellen, die Frequenzen in vorbestimmten Bereichen haben, im Mundraum ermöglichen.

Gemäß verschiedenen Aspekten kann eine Vorrichtung bereitgestellt werden, die eine Beißschiene (z.B. ein Mundstück) aufweist. Gemäß verschiedenen Aspekten kann die Vorrichtung zwei Beißschienen aufweisen. Beispielsweise kann eine erste Beißschiene der zwei Beißschienen für einen Oberkiefer-Teil des Mundraums geeignet sein. Beispielsweise kann eine zweite Beißschiene der zwei Beißschienen für einen Unterkiefer-Teil des Mundraums geeignet sein.

Gemäß verschiedenen Aspekten kann eine Vorrichtung bereitgestellt werden, die eine Steuervorrichtung (z.B. eine Ansteuerungselektronik) zum Steuern der Vorrichtung aufweist. Beispielsweise kann die Steuervorrichtung mittels Kabel und/oder kabellos (z.B. per Funk) mit einer Beißschiene der Vorrichtung verbunden sein. Somit kann beispielsweise eine schmalere Bauform erreicht werden, wodurch z.B. ein Tragekomfort erhöht werden kann.

Gemäß verschiedenen Aspekten wird eine Vorrichtung zum Reinigen der Zähne bereitgestellt, die nicht von einer manuellen Führung abhängig ist. Dadurch können Fehlerquellen in der Reinigung reduziert werden.

Gemäß verschiedenen Aspekten wird eine Vorrichtung bereitgestellt, die eine zeitsynchrone Reinigung aller Zähne und der dazu korrespondierenden Zahnzwischenräume innerhalb eines Bereichs eines Mundraums (z.B. eines Quadranten) und/oder des gesamten Mundraums ermöglicht.

Gemäß verschiedenen Aspekten kann das Ultraschallsystem verwendet werden, um eine Osseointegration von Implantaten zu verbessern (z.B. eine Heilung zu fördern). Beispielsweise kann eine an die Implantate angepasste Beißschienen-Einlage in das Ultraschallsystem eingesetzt werden.

Gemäß verschiedenen Aspekten kann das Ultraschallsystem verwendet werden, um eine Heilung nach einer Knochenaugmentation zu verbessern. Beispielsweise kann eine an eine (z.B. operierte) anatomische oder pathologische Struktur angepasste Beißschienen-Einlage in das Ultraschallsystem eingesetzt werden.

Gemäß verschiedenen Aspekten kann das Ultraschallsystem verwendet werden, um eine Knochenatrophie hinauszuzögern (z.B. zu verhindern).

Gemäß verschiedenen Aspekten wird ein Ultraschallsystem bereitgestellt, das bei Patienten mit Osteoradionekrosen und/oder Antiresorptiva-assoziierten Kiefernekrosen zur Zahnreinigung und/oder zur Regenerationsunterstützung von Strukturen innerhalb des Mundraums verwendet werden kann.

Gemäß verschiedenen Aspekten kann die Vorrichtung einen Zulauf und/oder einen Ablauf aufweisen, um ein Koppelmedium (z.B. eine Flüssigkeit und/oder ein Gel und/oder eine Zahnpasta) in den Mundraum einzubringen bzw. dieses aus dem Mundraum zu entfernen. Der Zulauf und/oder der Ablauf kann mit der Beißschiene gekoppelt sein, z.B. in die Beißschiene integriert sein.

Gemäß verschiedenen Aspekten wird ein Ultraschallsystem bereitgestellt, das aufweist: eine Beißschiene zum Einbringen und Positionieren in einer Mundhöhle, wobei die Beißschiene ein oder mehrere Ultraschallwandler zum Anlegen eines ausgewählten Ultraschallsignals an ein oder mehrere Strukturen innerhalb einer Mundhöhle (z.B. an mehrere Zähne und/oder zu mehreren Zähnen (z.B. direkt und/oder indirekt) angrenzende Strukturen, wie beispielsweise korrespondierende Interdentalräume, ein Zahnhalteapparat, benachbartes Gewebe (z.B. Zahnfleisch)) zum Reinigen und/oder zur Regeneration der ein oder mehreren Strukturen innerhalb der Mundhöhle aufweist.

Anschaulich können somit verschiedene Verschmutzungen mittels Ultraschallwellen entfernt werden. Insbesondere können leichte Anhaftungen (z.B. Biofilme, Speisereste), hartnäckige Verunreinigungen (z.B. Zahnstein) sowie im allgemeinen bakterielle Verunreinigungen von Zähnen, den korrespondierenden Interdentalräumen sowie dem Zahnfleisch durch den Einsatz von Schwingungen in verschiedenen, vorbestimmten Frequenzspektren entfernt werden.

Beispielsweise können die in der Beißschiene angebrachten Ultraschallwandler (z.B. die Ultraschallemitter) mittels einer gezielten Ausrichtung eines akustischen Feldes (z.B. eines Ultraschall(wellen)feldes) ein menschliches und/oder tierisches Gebiss vollständig, zeitsynchron und schonend reinigen. Beispielsweise kann die Beißschiene so ausgestaltet sein, dass sie leicht an das menschliche und/oder tierische Gebiss (z.B. eine individuelle Zahnstellung) angepasst werden kann. Somit kann beispielsweise ein höherer Tragekomfort und/oder eine Nutzung des Systems durch mehrere Nutzer ermöglicht werden. Beispielsweise kann die Reinigung der Zähne (z.B. der Zahnoberflächen), den korrespondierenden Interdentalräumen sowie dem Zahnfleisch ohne die Einwirkung von Bürsten (bzw. Borsten) möglich sein.

Beispielsweise kann eine Beißschiene des Ultraschallsystems eine Kommunikationsschnittstelle (z.B. für eine kabelgebundene und/oder eine kabellose Kommunikation) aufweisen. Somit kann die Beißschiene mit einer Steuervorrichtung (z.B. einem dafür eingerichteten Gerät, und/oder einem Gerät auf dem eine korrespondierende Steuerungs-App eingerichtet ist) verbunden werden. Beispielsweise kann die Steuervorrichtung eingerichtet sein zum Unterstützen des Benutzers bei der Anwendung durch audiovisuelle Signale (z.B. eine audiovisuelle Zahnputzbegleitung). Beispielsweise kann das Ultraschallsystem vor Staub und/oder Flüssigkeiten geschützt sein, sodass eine schnelle Reinigung ermöglicht wird. Beispielsweise kann das Ultraschallsystem eine geräuscharme Anwendung ermöglichen. Beispielsweise kann das Ultraschallsystem eine an die Zähne anpassbare Zahnschiene (z.B. eine Beißschienen-Einlage) aufweisen und somit einen hohen Tragekomfort für einen Nutzer ermöglichen. Beispielsweise kann das Ultraschallsystem separate Beißschienen für eine obere Zahnreihe und eine untere Zahnreihe aufweisen. Beispielsweise kann die anpassbare Zahnschiene (z.B. anpassbar an eine Bissform) zum Einlegen in die Beißschiene eingerichtet sein. Somit wird durch eine modulare Bauweise ermöglicht, die Beißschiene auszutauschen. Somit kann beispielsweise eine Reinigung von Kontaktflächen ermöglicht werden, an denen das Ultraschallsystem Kontakt zu dem Gebiss hat. Ferner kann somit eine hygienische Mehrpersonenbenutzung ermöglicht werden, da die Zahnschienen individuell eingelegt werden können.

Gemäß verschiedenen Aspekten kann ein Ultraschallsystem zum Reinigen und/oder zur Regeneration von ein oder mehreren Strukturen innerhalb einer Mundhöhle bereitgestellt werden. Die ein oder mehreren Strukturen innerhalb der Mundhöhle können mehrere Zähne und/oder zu den mehreren Zähnen direkt oder indirekt angrenzende Strukturen aufweisen (z.B. sein).

Verschiedene beispielhafte Ausführungsformen sind in den Figuren dargestellt und werden im Folgenden näher erläutert.
Figuren 1 bis 9 zeigen jeweils ein Ultraschallsystem gemäß verschiedenen Aspekten.
Figur 10 zeigt ein Verfahren zum Reinigen und/oder zur Regeneration von ein oder mehreren Strukturen innerhalb einer Mundhöhle gemäß verschiedenen Aspekten.

In der folgenden ausführlichen Beschreibung wird auf die beigefügten Zeichnungen Bezug genommen, die Teil dieser bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende Beschreibung ist deshalb nicht in einschränkendem Sinne aufzufassen, und der Schutzumfang der vorliegenden Erfindung wird durch die angefügten Ansprüche definiert.

Gemäß verschiedenen Aspekten können ein oder mehrere Ultraschallwellen bereitgestellt (z.B. erzeugt) werden. Es versteht sich, dass die ein oder mehreren Ultraschallwellen durch ein zu der jeweiligen Ultraschallwelle der ein oder mehreren Ultraschallwellen korrespondierendes Ultraschallsignal repräsentiert werden können. Daher kann hierin eine Ultraschallwelle auch mittels des korrespondierenden Ultraschallsignals bezeichnet werden und umgekehrt. Unter einer Ultraschallwelle kann die tatsächliche physikalische (Schall-)Welle verstanden werden, die sich innerhalb eines Mediums bzw. eines Stoffs ausbreiten kann. Unter einem Ultraschallsignal kann eine messtechnische und/oder anregungstechnische Entsprechung einer Ultraschallwelle verstanden werden, z.B. in Form von ein oder mehreren Charakteristika der Ultraschallwelle (z.B. als Messwert).

Jedes Ultraschallsignal kann Informationen (z.B. in Form von Daten) aufweisen, die eine dazu korrespondierende Ultraschallwelle repräsentiert, wie beispielsweise: eine Intensität, und/oder eine Frequenz, und/oder eine Phasenlange, und/oder eine Wellenlänge, und/oder einer Dauer (z.B. eine zeitliche Verzögerung), und/oder einen Startzeitpunkt, und/oder einen Endzeitpunkt von einer korrespondieren Ultraschall-Welle. Ferner können Ultraschallsignale gepulst sein. Anschaulich kann ein gepulstes Ultraschallsignal pro Puls mehrere Ultraschallwellen erzeugen, die ein oder mehrere vorbestimmte Frequenzen aufweisen. Zwischen zwei Pulsen kann eine Pause sein, in der keine Ultraschallwellen erzeugt werden. Eine Anzahl von Pulsen pro Zeiteinheit (z.B. pro Sekunde) kann als Pulsfrequenz bezeichnet werden. Es versteht sich, dass die Pulsfrequenz die Zeit zwischen einem jeweiligen Beginn zweier direkt aufeinanderfolgenden Ultraschallpulsen repräsentiert.

Beispielsweise kann auf Grundlage eines Ultraschallsignals die zum Ultraschallsignal korrespondierende Ultraschallwelle erzeugt werden (z.B. mittels eines Ultraschallwandlers (z.B. eines Ultraschall(wellen)emitters)). Beispielsweise kann auf Grundlage einer Ultraschallwelle das zu der Ultraschallwelle korrespondierende Ultraschallsignal erzeugt (z.B. ermittelt, gemessen, detektiert) werden (z.B. mittels eines Ultraschallwandlers (z.B. mittels eines Ultraschall(wellen)-empfängers)). Unter einem "Einkoppeln eines Ultraschallsignals in ein Medium oder einen Stoff" kann verstanden werden, dass in dem Medium bzw. in dem Stoff eine Ultraschallwelle erzeugt wird, die zu dem Ultraschallsignal korrespondiert.

Als ein Ultraschallwandler kann hierin ein Bauteil bezeichnet werden, das Ultraschallwellen in Ultraschallsignale umwandeln kann und umgekehrt. Beispielsweise kann ein Ultraschallwandler eingerichtet sein, um ein oder mehrere Ultraschallsignale in Form von ein oder mehreren Ultraschallwellen in ein Medium zu emittieren. Eine derartiger Ultraschallwandler kann beispielsweise ein oder mehrere Ultraschallemitter aufweisen (z.B. daraus bestehen). Beispielsweise kann ein Ultraschallwandler eingerichtet sein, um ein oder mehrere Ultraschallwellen innerhalb eines Mediums und/oder aus einem Medium zu empfangen und die empfangenen ein oder mehreren Ultraschallwellen in ein jeweils dazu korrespondierendes Ultraschallsignal umzuwandeln. Ein derartiger Ultraschallwandler kann beispielsweise ein oder mehrere Ultraschallempfänger aufweisen (z.B. daraus bestehen). Es versteht sich, dass ein Ultraschallwandler sowohl ein oder mehrere Ultraschallempfänger als auch ein oder mehrere Ultraschallemitter aufweisen kann.

Beispielsweise können ein oder mehrere Ultraschallwellen aus ein oder mehreren Ultraschallsignalen mittels ein oder mehrerer Ultraschallwandler (z.B. mittels ein oder mehrerer Ultraschallemitter) erzeugt werden. Beispielsweise können ein oder mehrere Ultraschallwellen basierend auf dem piezoelektrischen Effekt erzeugt werden, z.B. mittels eines piezoelektrischen Stoffes oder eines piezoelektrischen Kristalls. Beispielsweise können ein oder mehrere Ultraschallwellen aus ein oder mehreren dazu korrespondierenden Ultraschallsignalen mittels des piezoelektrischen Effektes erzeugt werden. Beispielsweise können ein oder mehrere Ultraschallwellen als ein oder mehrere korrespondierende Ultraschallsignale mittels ein oder mehrerer Ultraschallwandler (z.B. mittels ein oder mehrerer Ultraschallempfänger) empfangen werden. Beispielsweise können die ein oder mehreren empfangenen Ultraschallwellen in ein oder mehrere dazu korrespondierende Ultraschallsignale umgewandelt werden. Unter Erzeugen von Ultraschallsignalen oder Ermitteln von Ultraschallsignalen kann ein Vorgang verstanden werden, der ein Empfangen (z.B. Erfassen) von ein oder mehreren Ultraschallwellen und ein Umwandeln der empfangenen ein oder mehreren Ultraschallwellen in die dazu korrespondierenden Ultraschallsignale umfassen kann. Beispielsweise können ein oder mehrere Ultraschallwandler dazu eingerichtet sein, Ultraschallsignale zu ermitteln. Beispielsweise können Ultraschallwellen basierend auf dem piezoelektrischen Effekt in Ultraschallsignale umgewandelt werden, z.B. mittels eines piezoelektrischen Stoffes oder eines piezoelektrischen Kristalls.

Für die Ausbreitung (z.B. eine Geschwindigkeit, eine Strecke) von Ultraschallwellen in einem Stoff kann die Impedanz, d.h. der Widerstand, der der Ausbreitung von Wellen entgegenwirkt, von Bedeutung sein. Insbesondere kann eine Ultraschallwelle an Grenzflächen zwischen zwei benachbarten Stoffen (zumindest) teilweise umgelenkt (z.B. reflektiert) und/oder teilweise transmittiert werden. Der jeweilige umgelenkte bzw. transmittierte Anteil ist von einem Impedanz-Unterschied an der Grenzfläche der zwei benachbarten Stoffe abhängig.

Je größer der Impedanz-Unterschied an der Grenzfläche der zwei aneinander angrenzenden Stoffe ist, desto größer (verglichen mit dem transmittierten Anteil) kann der Anteil der umgelenkten Ultraschallwelle sein. An einer Grenzfläche von zwei Stoffen mit großem Impedanz-Unterschied (z.B. mehr als 25 %, 50 %, 75 % oder mehr als 100 %) kann eine auftreffende Ultraschallwelle stark umgelenkt werden (z.B. zu mehr als 10 %, 25 %, 50 %, 75 % oder zu mehr als 95 %). Beispielsweise kann dieser Unterschied zwischen einem Gas (z.B. Luft) und einer Flüssigkeit (z.B. Wasser) besonders stark ausgeprägt sein.

Je geringer der Impedanz-Unterschied an der Grenzfläche der zwei aneinander angrenzenden Stoffe ist, desto geringer (verglichen mit dem transmittierten Anteil) kann der Anteil der umgelenkten Ultraschallwelle ausgeprägt sein. Ferner kann eine Laufrichtung des umgelenkten Anteils der Ultraschallwelle aufgrund des hohen Impedanz-Unterschieds beim Übergang von einem ersten Stoff in einen zweiten Stoff der zwei aneinander angrenzenden Stoffe stärker verändert werden als bei einem geringeren Impedanz-Unterschied.

Gemäß verschiedenen Aspekten kann ein Koppelmedium verwendet werden, das die Entstehung von Luftblasen verhindert. Beispielsweise kann das Koppelmedium verwendet werden, um einen mit einem Gas gefüllten Hohlraum zu füllen. Somit kann eine Ultraschall-Wellenleitung innerhalb des Hohlraums ermöglicht werden. Beispielsweise kann das Koppelmedium eingerichtet sein, dass eine Intensität einer Ultraschallwelle von dem Koppelmedium zu weniger als 1% reduziert wird, bei einer einfachen Laufstrecke von 6 cm. Beispielsweise kann das Koppelmedium Wasser sein und somit eine Intensität der Ultraschallwelle um 50% bei einer Laufstrecke von 6 m reduzieren. Beispielsweise kann das Koppelmedium einen Absorptionskoeffizienten von Ultraschallwellen von 1 dB/m oder weniger aufweisen. Es versteht sich, dass eine Absorption von Ultraschallwellen Frequenzabhängig ist und sich die genannten Werte auf den für eine Anwendung relevanten Frequenzbereich beziehen. Eine möglichst geringe Absorption des Koppelmediums kann einen optimalen Energieverbrauch des Ultraschallsystems ermöglichen.

Gemäß verschiedenen Aspekten kann das Koppelmedium ein oder mehrere Zusätze aufweisen, wie beispielsweise zusätzliche Inhaltsstoffe, die dazu geeignet sind, um Regenerationsprozesse (z.B. Remineralisierungsprozesse) zu fördern (z.B. kann das Koppelmedium dazu Fluor und/oder Hydroxylapatit und/oder Zinkoxid aufweisen) und/oder um Reinigungsprozesse zu fördern (z.B. kann das Koppelmedium dazu Putzkörper aufweisen). Als Putzkörper können Zusätze einer Zahnpasta bezeichnet werden, die zusammen mit einer Zahnbürste Verunreinigungen (z.B. Plaque und/oder schädliche Bakterien) von einer Zahnoberfläche entfernen können. Gemäß verschiedenen Aspekten können die Putzkörper in einem Koppelmedium auf ein oder mehrere Ultraschallwellen angepasst werden, z.B. in dem sie Kavitationseffekte bei bestimmten Frequenzen unterstützen.

Gemäß verschiedenen Aspekten kann eine Laufzeit einer Ultraschallwelle ermittelt werden. Eine Ultraschallwelle kann an einem Startpunkt emittiert werden. Die Ultraschallwelle breitet sich innerhalb eines Mediums mit einer Ausbreitungsgeschwindigkeit aus (z.B. mit Schallgeschwindigkeit). Die Ausbreitungsgeschwindigkeit kann von dem Medium abhängig sein. Die Ausbreitungsgeschwindigkeit kann beispielsweise durch die Impedanz repräsentiert werden. Die Laufzeit kann beispielsweise auf Grundlage der Ausbreitungsgeschwindigkeit der Ultraschallwelle innerhalb des Mediums und der Laufzeit ermittelt werden.

Gemäß verschiedenen Aspekten können ein oder mehrere Ultraschallsignale bereitgestellt (z.B. erzeugt) werden. Als Ultraschall können ein oder mehrere (Schall-)Wellen bezeichnet werden, die jeweils eine Frequenz zwischen 20 kHz und 10 GHz haben. Beispielsweise kann eine Ultraschallwelle eine Frequenz zwischen 1 und 40 MHz aufweisen (z.B. haben). Beispielsweise kann eine Ultraschallwelle eine Intensität (z.B. Strahlungsintensität, Schallintensität) zwischen 5 mW/cm² und 125 mW/cm² aufweisen.

Gemäß verschiedenen Aspekten wird ein Ultraschallsystem bereitgestellt, welches ein oder mehrere Funktionalitäten aufweisen kann. Die jeweiligen Funktionalitäten der ein oder mehreren Funktionalitäten können auf physikalischen Effekten des Ultraschalls beruhen. Beispielsweise können die ein oder mehreren Funktionalitäten ein oder mehrere der nachfolgend genannten Funktionalitäten aufweisen (z.B. sein). Insbesondere können die jeweiligen Funktionalitäten von voneinander verschiedenen Ultraschallsignalen von einer bestimmten Kombination von Ultraschallfrequenzen, Intensitäten und Pulsfrequenzen der Ultraschallsignale (bzw. der dazu korrespondierenden Ultraschallwellen) abhängig sein, wie nachfolgend beschrieben wird.

Beispielsweise kann eine der ein oder mehreren Funktionalitäten eine Reinigung der Zahnoberflächen (z.B. ein Entfernen von Biofilm und/oder Zahnstein) mittels einer Kavitationswirkung sein. Beispielsweise kann die Kavitationswirkung durch ein geeignetes Koppelmedium (z.B. eine geeignete Zahnpasta) verbessert werden. Ferner kann die Reinigungswirkung durch eine Kombinationswirkung mit dem Koppelmedium, z.B. aufgrund von Putzkörpern innerhalb des Koppelmediums verstärkt werden.

Beispielsweise kann eine der ein oder mehreren Funktionalitäten eine Reinigung der Zahnzwischenräume (bzw. Interdentalräume) sein. Beispielsweise können Biofilme, Zahnstein und andere Verunreinigungen mittels Kavitation entfernt werden.

Beispielsweise kann eine der ein oder mehreren Funktionalitäten eine antibakterielle Oberflächenbehandlung sein. Beispielsweise kann eine Desinfektion durch Ultraschall und/oder durch Licht (z.B. Ultraviolettes Licht) durchgeführt werden.

Beispielsweise kann eine Desinfektion durch Verwenden physikalischer Effekte durchgeführt werden. Beispielsweise können dazu Ultraschallwellen verwendet werden, die Frequenzen zwischen 1 kHz und 500 kHz, z.B. beispielsweise 5 kHz und 300 kHz oder beispielsweise 20 kHz und 200 kHz oder beispielsweise zwischen 20 kHz und 60 kHz aufweisen. Beispielsweise können mehrere Ultraschallwellen mit voneinander verschiedenen Frequenzen überlagert werden, um eine effektivere Desinfektion durchzuführen. Ferner können derartige Ultraschallwellen Intensitäten zwischen 1 mW/cm² und 100 W/cm² oder beispielsweise zwischen 5 mW/cm² und 50 W/cm², oder beispielsweise zwischen 0,1 W/cm² und 10 W/cm² aufweisen. Beispielsweise kann durch einen kleineren Frequenzbereich und/oder geringere Intensitäten Energie weniger Energie verbraucht werden und eine schonendere Reinigung erzielt werden. Beispielsweise kann eine Anwendungsdauer in einem Bereich von ein oder mehreren Minuten liegen. Beispielsweise kann eine Anwendungsdauer weniger als 10 min (z.B. weniger als 5 min, z.B. weniger als 2 min betragen).

In einem Beispiel, können mehrere Ultraschallwellen zur Desinfektion verwendet werden, die jeweils eine Frequenz aus einem Frequenzbereich zwischen 20 kHz und 60 kHz aufweisen können. Beispielsweise haben voneinander verschiedene Ultraschallwellen verschiedene Frequenzen. Beispielsweise können die ein oder mehreren Ultraschallwellen jeweils eine Leistung von ca. 100 W (z.B. 100 W/cm²) aufweisen.

Beispielsweise kann eine Desinfektion durch Verwenden chemischer Effekte durchgeführt werden. Beispielsweise können dazu Ultraschallwellen verwendet werden, die Frequenzen zwischen 50 kHz und 5 MHz oder beispielsweise 100 kHz und 2 MHz oder beispielsweise zwischen 200 kHz und 1 MHz aufweisen. Beispielsweise können mehrere Ultraschallwellen mit voneinander verschiedenen Frequenzen überlagert werden, um eine effektivere Desinfektion durchzuführen. Ferner können derartige Ultraschallwellen Intensitäten zwischen 1 mW/cm² und 100 W/cm² oder beispielsweise zwischen 5 mW/cm² und 50 W/cm² oder beispielsweise zwischen 0,1 W/cm² und 10 W/cm² aufweisen. Beispielsweise kann eine Anwendungsdauer in einem Bereich von ein oder mehreren Minuten liegen. Beispielsweise kann eine Anwendungsdauer weniger als 10 min (z.B. weniger als 5 min, z.B. weniger als 2 min betragen).

Beispielsweise kann eine der ein oder mehreren Funktionalitäten eine Anregung eines Regenerationsverhaltens von Strukturen innerhalb eines Mundraums (z.B. von ein oder mehreren Zähnen (z.B. durch eine Remineralisierung), eines Zahnhalteapparats (z.B. vom Zahnfleisch) und/oder eines Kieferknochens (z.B. aufgrund einer Stimulation einer Zell-Proliferation) aufgrund ultraschallbasierter Stimulation sein.

Beispielsweise können gepulste und/oder kontinuierlich ausgesendete (d.h. nicht gepulste) Ultraschallwellen mit bestimmten Frequenzen geeignet sein, um eine Osteoblasten-Proliferation (was zu einer schnelleren Knochenfrakturheilung führen kann) zu stimulieren.

Kontinuierlich ausgesendete Ultraschallwellen zum Stimulieren einer Osteoblasten-Proliferation können Frequenzen zwischen 30 kHz und 60 kHz aufweisen oder beispielsweise zwischen 40 kHz und 50 kHz aufweisen oder beispielsweise von 45 kHz aufweisen. Kontinuierlich ausgesendete Ultraschallwellen zum Stimulieren einer Osteoblasten-Proliferation können eine Leistung zwischen 10 mW/cm² und 40 mW/cm² aufweisen oder beispielsweise zwischen 20 mW/cm² und 30 mW/cm² aufweisen oder beispielsweise von 25 mW/cm² aufweisen.

Gepulste Ultraschallwellen zum Stimulieren einer Osteoblasten-Proliferation können Frequenzen zwischen 45 kHz und 2 MHz aufweisen oder beispielsweise zwischen 500 kHz und 1,5 MHz aufweisen oder beispielsweise von 1 MHz aufweisen. Gepulste ausgesendete Ultraschallwellen zum Stimulieren einer Osteoblasten-Proliferation können eine Leistung zwischen 100 mW/cm² und 400 mW/cm² aufweisen oder beispielsweise zwischen 200 mW/cm² und 300 mW/cm² aufweisen oder beispielsweise von 250 mW/cm² aufweisen. Gepulste Ultraschallwellen zum Stimulieren einer Osteoblasten-Proliferation können eine Pulsfrequenz zwischen 50 Hz und 100 Hz aufweisen oder beispielsweise zwischen 60 Hz und 70 Hz oder beispielsweise von 63 Hz aufweisen. Gepulste Ultraschallwellen zum Stimulieren einer Osteoblasten-Proliferation können eine Pulsdauer zwischen 1 ms und 20 ms aufweisen oder beispielsweise zwischen 1 ms und 5 ms oder beispielsweise von rund 3 ms (z.B. 3,2 ms) aufweisen.

Beispielsweise können Ultraschallwellen mit bestimmten Frequenzen geeignet sein, um eine Proliferation von Odontoblast-ähnlichen Zellen zu stimulieren, beispielsweise in dem eine Produktion von proliferationsassoziierten Proteinen stimuliert wird (z.B. hsp25/27, TGFb1, VEGF). Beispielsweise können gepulste Ultraschallwellen zum Stimulieren einer Proliferation von Odontoblast-ähnlichen Zellen aus dem LIPUS (Low Intensity Pulsed UltraSound)-Bereich verwendet werden.

Ultraschallwellen aus dem LIPUS-Bereich können Frequenzen zwischen 0,5 MHz und 2 MHz aufweisen, beispielsweise zwischen 1 MHz und 1,5 MHz aufweisen. Ultraschallwellen aus dem LIPUS-Bereich können eine Leistung zwischen 1 mW/cm² und 1000 mW/cm² aufweisen, beispielsweise zwischen 10 mW/cm² und 750 mW/cm² aufweisen oder beispielsweise von 20 mW/cm² und 500 mW/cm² aufweisen. Ultraschallwellen aus dem LIPUS-Bereich können eine Pulsfrequenz zwischen 0,1 kHz und 10 kHz aufweisen, beispielsweise zwischen 0,5 kHz und 5 kHz oder beispielsweise von 1 kHz aufweisen. Ultraschallwellen aus dem LIPUS-Bereich können eine Pulsdauer zwischen 1 µs und 1 ms aufweisen, beispielsweise zwischen 50 µs und 500 µs oder beispielsweise von rund 200 µs aufweisen.

Beispielsweise kann ein gepulster Ultraschall verwendet werden, um eine Parodontal-Ligament-Regeneration (z.B. ein Gewebe, das den Zahn an einem korrespondierenden Knochen befestigt) nach einem Parodonthaltherapie (PA)-Lappenchirurgie-Eingriff zu beschleunigen.

Gepulste Ultraschallwellen zum Beschleunigen einer Parodontal-Ligament-Regeneration können Frequenzen zwischen 100 kHz und 5 MHz aufweisen, beispielsweise zwischen 500 kHz und 2 MHz aufweisen oder beispielsweise von 1,5 MHz aufweisen. Gepulste ausgesendete Ultraschallwellen zum Beschleunigen einer Parodontal-Ligament-Regeneration können eine Leistung zwischen 1 mW/cm² und 500 mW/cm² aufweisen, beispielsweise zwischen 10 mW/cm² und 100 mW/cm² aufweisen oder beispielsweise von 30 mW/cm² aufweisen. Gepulste Ultraschallwellen zum Beschleunigen einer Parodontal-Ligament-Regeneration können eine Pulsfrequenz zwischen 0,1 kHz und 10 kHz aufweisen, beispielsweise zwischen 0,5 kHz und 5 kHz oder beispielsweise von 1 kHz aufweisen. Gepulste Ultraschallwellen zum Beschleunigen einer Parodontal-Ligament-Regeneration können eine Pulsdauer zwischen 0,1 ms und 10 ms aufweisen, beispielsweise zwischen 0,5 ms und 5 ms oder beispielsweise von rund 3 ms aufweisen.

Beispielsweise kann ein gepulster Ultraschall verwendet werden, um eine bessere Osseointegration von Implantaten zu ermöglichen. Beispielsweise können gepulste Ultraschallwellen zum Ermöglichen einer besseren Osseointegration von Implantaten aus dem LIPUS (Low Intensity Pulsed UltraSound)-Bereich verwendet werden.

Beispielsweise können Ultraschallwellen mit bestimmten Frequenzen geeignet sein, um eine Remineralisierung des Dentins mittels Hoch-Intensiv-Fokussierter Ultraschall (HIFU) zu ermöglichen.

Ultraschallwellen aus dem HIFU-Bereich können Frequenzen zwischen 0,5 MHz und 10 MHz aufweisen, beispielsweise zwischen 1 MHz und 5 MHz aufweisen. Ultraschallwellen aus dem HIFU-Bereich können eine Leistung zwischen 0,5 kW/cm² und 20 kW/cm² aufweisen, beispielsweise zwischen 1 kW/cm² und 10 kW/cm² aufweisen oder beispielsweise von rund 5 kW/cm² aufweisen (z.B. mit einer Gesamtbestrahlungszeit von weniger als 3 Sekunden). Aufgrund einer derartig hohen Energiedichte und einer damit verbundenen Temperaturerhöhung, sollten Ultraschallwellen aus dem HIFU-Bereich nur über sehr kurze Zeiträume (beispielsweise weniger als 1 ms oder beispielsweise weniger als 10 µs oder beispielsweise weniger als 1 µs) angewendet werden.

Beispielsweise können Ultraschallwellen aus dem HIFU-Bereich geeignet sein, um Zähne, insbesondere die jeweiligen Zahnoberflächen, zu remineralisieren. Eine Remineralisierung kann insbesondere durch ein Koppelmedium unterstützt werden, das Fluorid und/oder Hydroxalapatit aufweist.

Gemäß verschiedenen Aspekten werden hierin Verfahren und Vorrichtungen zum Reinigen und/oder zur Regeneration von ein oder mehreren Strukturen innerhalb einer Mundhöhle bereitgestellt. Als ein oder mehrere Strukturen innerhalb einer (z.B. der) Mundhöhle können hierin ein oder mehrere Zähne und/oder zu den mehreren Zähnen angrenzende Strukturen verstanden werden. Diese angrenzenden Strukturen können beispielsweise Strukturen des Zahnhalteapparates (wie z.B. Zahnfleisch, Kieferknochen (z.B. Unterkieferknochen, Oberkieferknochen)), Nerven und/oder andere Strukturen sein, die in direktem oder indirektem Kontakt zu einem oder mehreren Zähnen stehen. Ferner umfassen die ein oder mehrere Strukturen innerhalb der Mundhöhle auch zu den jeweiligen Strukturen zugehörige Oberflächen (z.B. Zahnoberflächen, Knochenoberflächen, Zahnfleischoberflächen) und/oder Zwischenräume (z.B. Interdentalräume, Zahnzwischenräume, ein Raum zwischen Zahnfleisch und Zahnhals etc.) zwischen jeweils direkt zueinander benachbarten Strukturen der ein oder mehreren Strukturen innerhalb der Mundhöhle.

Gemäß verschiedenen Aspekten wird ein Ultraschallsystem bereitgestellt, welches dazu eingerichtet ist, Verunreinigungen und/oder pathologische Veränderungen zu ermitteln. Beispielsweise kann das Ultraschallsystem ermitteln, ob Karies vorhanden ist, z.B. mittels einer Lichttransmission und/oder einer fotooptischen Transillumination (z.B. Faseroptische Transillumination). Beispielsweise kann das Ultraschall ermitteln, ob Zahnstein und/oder Parodontitis vorliegt. Gemäß verschiedenen Aspekten kann das Ultraschallsystem eingerichtet sein, ein zum Ermittlungsergebnis passendes Ultraschallsignal auszuwählen, um die ermittelte Verunreinigung zu entfernen und/oder einen Regenerationsprozess zu unterstützen.

Gemäß verschiedenen Aspekten kann das Ultraschallsystem geeignet sein (z.B. verwendet werden), um nach Zahntraumen, und/oder Kieferknochenfrakturen eine Regeneration zu verbessern (z.B. zu beschleunigen). Beispielsweise kann eine anpassbare Zahnschiene eingerichtet sein, um regelmäßig an den jeweiligen Regenerationszustand angepasst zu werden.

Die beschriebenen Verfahren und Bauteile können jedoch zur Ermittlung von Messdaten und unter Umständen auch Erkenntnissen über einen Menschen oder über ein Tier dienen. Eventuell könnte eine Behandlung mittels hierin beschriebener Aspekte unterstützt werden, eine Heilbehandlung oder Diagnose als solche sollen hierdurch jedoch nicht erfasst werden.

Im Folgenden werden verschiedene beispielhafte Ausführungsformen eines Ultraschallsystems gemäß verschiedenen Aspekten anhand der beigefügten Figuren erläutert.

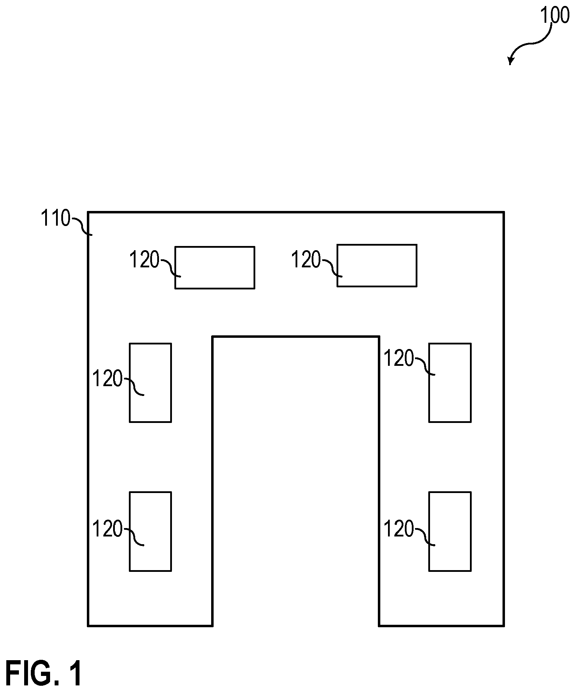

**Fig.1** veranschaulicht schematisch ein Ultraschallsystem 100 gemäß verschiedenen Aspekten. Das Ultraschallsystem 100 kann eine Beißschiene 110 aufweisen, die zum Einbringen und Positionieren in einer Mundhöhle eines Menschen und/oder eines Tieres eingerichtet ist. Anschaulich ist eine derartige Beißschiene an geometrischen Dimensionen einer menschlichen bzw. tierischen Mundhöhle angepasst.

Insbesondere kann die Beißschiene an die geometrischen Dimensionen von Mundhöhlen von Erwachsenen, Adoleszenten und/oder Kindern angepasst sein.

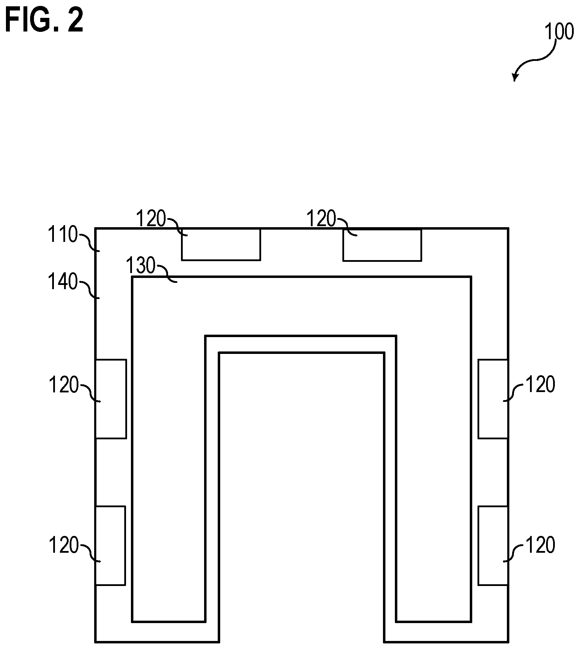

**Fig.2** veranschaulicht schematisch ein Ultraschallsystem 100 gemäß verschiedenen Aspekten, das eine Beißschienen-Einlage 130, aufweist, die an eine Zahnform eines Nutzers angepasst ist/werden kann. Gemäß verschiedenen Aspekten kann die Beißschiene 110 eine Beißschienen-Hülle 140 und die Beißschienen-Einlage 130 aufweisen. Die Beißschienen-Einlage 130 kann beispielsweise einen thermoplastischen Werkstoff aufweisen (z.B. im Wesentlichen daraus bestehen), so dass ihre Oberfläche an einen Zahnabdruck eines Nutzer angepasst werden kann. Beispielsweise kann die Beißschienen-Einlage 130 erhitzt werden, anschließend kann der Zahnabdruck des Nutzers erstellt werden. Im Anschluss, wenn die Beißschienen-Einlage 130 erkaltet ist, kann sie formstabil den Zahnabdruck des Nutzers darstellen. Somit kann ein minimaler Abstand zwischen der Beißschienen-Einlage 130 (und damit der Beißschiene 110) zu den Zähnen eines Nutzers ermöglicht werden, wodurch ein Effekt der Ultraschallwellen (z.B. Reinigungseffekt, Regenerationseffekt) verstärkt werden kann.

Ferner ermöglicht die Beißschienen-Einlage 130, dass mehrere Nutzer sich ein Ultraschallsystem 100 gemäß verschiedenen Aspekten teilen können, z.B. in dem jeder Nutzer seine eigene Beißschienen-Einlage 130 hat.

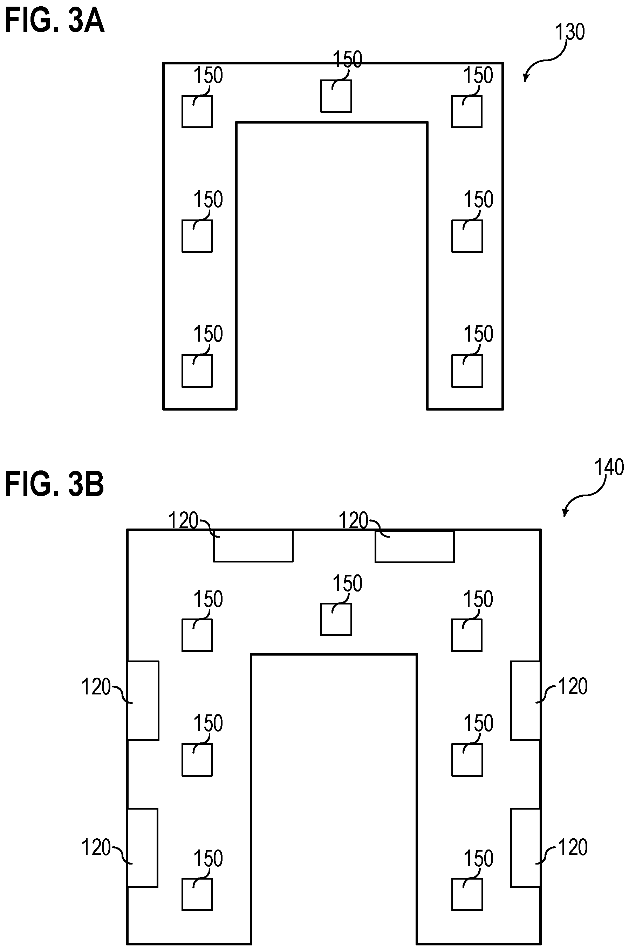

**Fig.3A und 3B** veranschaulichen schematisch ein Ultraschallsystem 100 gemäß verschiedenen Aspekten, wobei die Beißschienen-Einlage 130 (Fig. 3A) aus der Beißschienen-Hülle 140 (Fig. 3B) entfernt wurde. Gemäß verschiedenen Aspekten kann das Ultraschallsystem 100 mehrere ein oder mehrere Befestigungselemente 150 aufweisen, die eingerichtet sind, um die Beißschienen-Einlage 130 an der Beißschienen-Hülle 140 zu befestigen. Beispielsweise kann jedes der ein oder mehreren Befestigungselemente 150 einen ersten Teil und einen zum ersten Teil korrespondierenden zweiten Teil aufweisen. Beispielsweise kann der erste Teil an der Beißschienen-Einlage 130 und der zweite Teil an der Beißschienen-Hülle 140 angebracht sein. Beispielsweise können der erste und der zweite Teil der ein oder mehreren Befestigungselemente 150 mittels einer magnetischen Kraft einander haften. Beispielsweise können der erste und der zweite Teil der ein oder mehreren Befestigungselemente 150 mittels einer mechanischen Kraft einander haften (z.B. aufgrund eines Steckmechanismus). Beispielsweise können die ein oder mehreren Befestigungselemente 150 einen Fixierelement zum Fixieren der Beißschienen-Einlage 130 in der Beißschienen-Hülle 140 aufweisen.

Gemäß verschiedenen Aspekten kann die Beißschiene 110 ein oder mehrere Ultraschallwandler 120 (z.B. Ultraschallemitter) zum Anlegen von ein oder mehreren Ultraschallsignalen an ein oder mehrere Strukturen innerhalb einer Mundhöhle. Die mehreren Ultraschallsignale können zum Reinigen und/oder zur Regeneration der ein oder mehreren Strukturen innerhalb der Mundhöhle geeignet sein. Hierbei kann sich die jeweilige konkrete Eignung eines der mehreren Ultraschallsignale durch seine Frequenz, Intensität und/oder seine Pulsfrequenz (d.h. wenn es ein gepulstes Ultraschallsignal ist) ergeben.

Im Detail können die Ultraschallwandler 120 eingerichtet sein, ein vorbestimmtes Ultraschallsignal (z.B. das von einer Steuervorrichtung bereitgestellt wird) in mehrere Ultraschallwellen umzuwandeln. Somit kann, wenn das Ultraschallsystem in einem Mundraum positioniert ist, ein Ultraschallwellenfeld erzeugt werden. Innerhalb dieses Feldes können starke Druckschwankungen entstehen, welche wiederrum Kavitationen, d.h. ein Bilden und Auflösen von dampfgefüllten Hohlräumen (anschaulich Dampfblasen) in Flüssigkeiten (z.B. im Koppelmedium), auslösen können. Durch die Kavitation kann ein mechanischer Reinigungseffekt an festen (z.B. nicht flüssigen, nicht gasförmigen) benachbarten Strukturen entstehen. Im Mundraum können so beispielsweise Speisereste, Biofilme, Zahnstein oder andere Verunreinigungen von Zähnen (d.h. Zahnoberflächen und/oder Zahnzwischenräumen) gelöst werden. In analoger Weise können somit auch Verunreinigungen von anderen Strukturen innerhalb des Mundraums, die keine Zähne sind (z.B. vom Zahnfleisch), gelöst und somit entfernt werden.

Gemäß verschiedenen Aspekten können für voneinander verschiedene Verunreinigungen voneinander verschiedene Ultraschallsignale bereitgestellt werden, d.h. Ultraschallsignale, die sich in ihren jeweiligen Ultraschallfrequenzen, Intensitäten und/oder Puls-Eigenschaften unterscheiden.

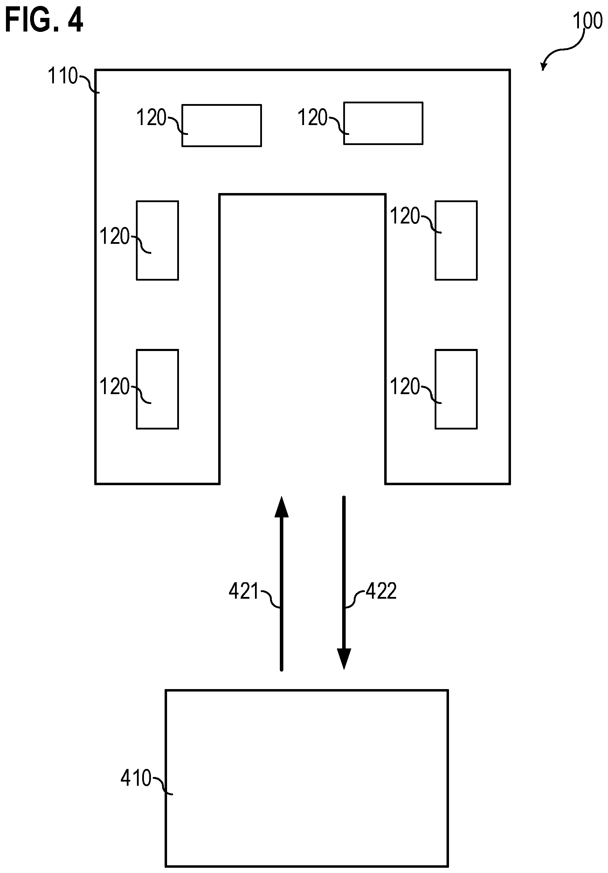

**Fig.4** veranschaulicht schematisch ein Ultraschallsystem 100 gemäß verschiedenen Aspekten, das eine Steuervorrichtung 410 zum Steuern des Ultraschallsystems 100 aufweist.

Gemäß verschiedenen Aspekten kann die Steuervorrichtung 410 eingerichtet sein, Signale an die Beißschiene 110, insbesondere an die darin enthaltenen Ultraschallwandler, zu übermitteln, dargestellt durch den Pfeil 421, und/oder von der Beißschiene 110 zu empfangen, dargestellt durch den Pfeil 422. Beispielsweise kann die Steuervorrichtung 410 zum Übermitteln von Signalen an die Beißschiene bzw. zum Empfangen von Signalen von der Beißschiene 110 eine Kabelverbindung und/oder eine kabellose Verbindung aufweisen. Es versteht sich, dass bei einer kabellosen Verbindung die Beißschiene und die Steuerungsvorrichtung jeweils eine Übermittlungs-Empfänger-Einheit aufweisen. Ferner versteht es sich, dass bei einer kabellosen Verbindung die Steuervorrichtung 410 und die Beißschiene 110 jeweils einen separaten Energiespeicher (z.B. in Form eines Akkumulators, einer Batterie) aufweisen. Bei einer kabelgebundenen Verbindung können sich die Beißschiene 110 und die Steuervorrichtung 410 einen gemeinsamen Energiespeicher teilen.

Gemäß verschiedenen Aspekten kann die Steuervorrichtung 410 durch eine App realisiert werden, die beispielsweise auf einem mobilen Endgerät (z.B. einem Smartphone, Tablet, Laptop etc.) installiert ist. Diese kann beispielsweise mittels einer kabellosen Verbindung (z.B. NFC, Bluetooth, WLAN, Infrarot etc.) mit der Beißschiene 110 gekoppelt sein. Es versteht sich jedoch, dass die Steuervorrichtung 410 auch ein separates einzelnes Gerät sein kann.

Gemäß verschiedenen Aspekten kann die Steuervorrichtung 410 einen Speicher aufweisen, auf dem mehrere voneinander verschiedene Ultraschallsignale gespeichert sind. Die Steuervorrichtung 410 kann eingerichtet sein, in Abhängigkeit von einer Nutzerauswahl, ein jeweiliges ausgewähltes Signal der mehreren voneinander verschiedenen Ultraschallsignale an die Beißschiene 110 insbesondere an die ein oder mehreren Ultraschallwandler 120 zu übermitteln. Die Beißschiene 110 kann eingerichtet sein, das von der Steuervorrichtung 410 übermittelte Ultraschallsignal zu empfangen und an die ein oder mehreren Ultraschallwandler 120 weiterzuleiten. Die ein oder mehreren Ultraschallwandler können eingerichtet sein, das empfangene Ultraschallsignal in eine dazu korrespondierende Ultraschallwelle umzuwandeln und somit ein Ultraschallwellenfeld innerhalb des Mundraums zu erzeugen.

Es versteht sich, dass in analoger Weise die Beschallung beendet werden kann und/oder ein anderes Signal ausgewählt werden kann.

Ferner versteht sich, dass ebenso eine Reihe (z.B. eine Serie, eine Auswahl) mehrerer Ultraschallsignale der mehreren voneinander verschiedenen Ultraschallsignale ausgewählt werden kann. Somit kann beispielsweise ein komplettes Reinigungsprogramm und/oder Regenerationsprogramm ermöglicht werden.

Gemäß verschiedenen Aspekten kann die Steuervorrichtung 410 eingerichtet sein, selbstständig ein passendes Ultraschallsignal aus den mehreren voneinander verschiedenen Ultraschallsignalen auszuwählen.

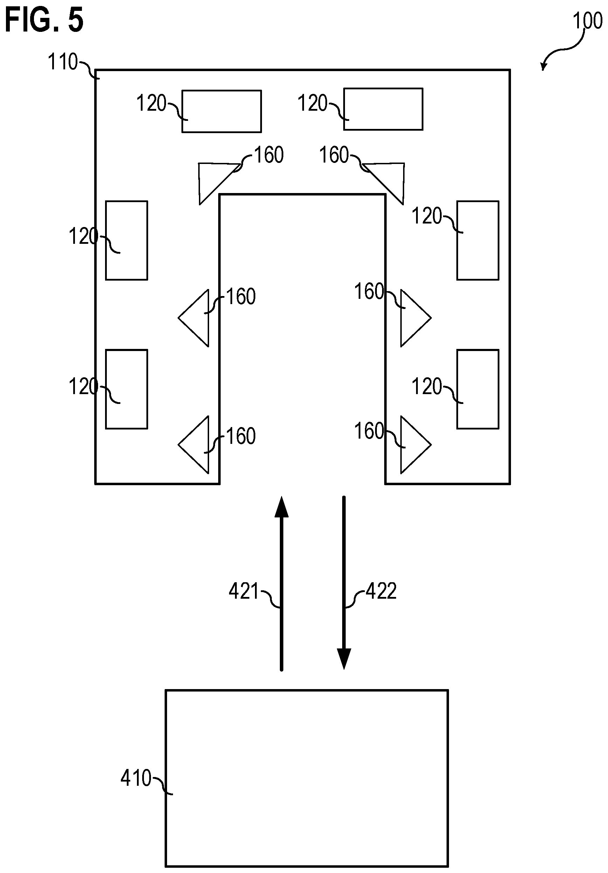

**Fig.5** veranschaulicht schematisch ein Ultraschallsystem 100 gemäß verschiedenen Aspekten, das ein oder mehrere Sensoren 160 aufweist. Die ein oder mehreren Sensoren können eingerichtet sein, ein oder mehrere Eigenschaften der ein oder mehreren Strukturen innerhalb der Mundhöhle zu ermitteln. Eine Eigenschaft kann Beispielsweise sein: ein Vorhandensein einer Verunreinigung, ein Grad (z.B. eine Ausprägung) einer Verunreinigung, eine Art einer Verunreinigung (z.B. Zahnstein, Speisereste, Biofilm, sonstige bakterielle Verunreinigungen), ein Vorhandensein einer pathologischen Veränderung (z.B. Karies, Demineralisierungen, Blutungen etc.), ein Grad der pathologischen Veränderung etc.

Die ein oder mehreren Sensoren 160 können eingerichtet sein, ein vorbestimmtes Sensorsignal zu emittieren und dieses anschließend als ein Messsignal zu empfangen. Das empfangene Messsignal kann sich im Vergleich zum emittierten Sensorsignal unterscheiden. Der Unterschied kann ein, mehrere oder alle der ein oder mehreren Eigenschaften der ein oder mehreren Strukturen innerhalb der Mundhöhle repräsentieren, welche durch eine Auswertung des Unterschieds mittels der Steuervorrichtung 410 ermittelt werden können.

Beispielsweise können die ein oder mehreren Sensoren 160 Ultraschallempfänger aufweisen (z.B. sein), die eingerichtet sind, von den Ultraschallwandlern 120 emittierte Ultraschallwellen zu empfangen und in ein Messsignal umzuwandeln. Das Messsignal kann ein, mehrere oder alle der ein oder mehreren Eigenschaften der ein oder mehreren Strukturen innerhalb der Mundhöhle repräsentieren. Beispielsweise können dazu in der Steuervorrichtung ein oder mehrere vorbestimmte Sensor-Ultraschallsignale bereitgestellt werden (z.B. gespeichert sein), die an die ein oder mehreren Ultraschallwandler 120 übermittelt werden können. Die ein oder mehreren vorbestimmten Sensor-Ultraschallsignale können beispielsweise zum Ermitteln von bestimmten Eigenschaften geeignet sein. Beispielsweise kann ein erstes Sensor-Ultraschallsignal zum Ermitteln eines Vorhandenseins einer Verunreinigung (z.B. von Zahnstein) geeignet sein. Beispielsweise kann ein zweites Sensor-Ultraschallsignal zum Ermitteln eines Grads einer pathologischen Veränderung (z.B. einer Parodontitis) geeignet sein. Beispielsweise können sich das erste und das zweite Sensor-Ultraschallsignal voneinander unterscheiden. Somit kann das von den ein oder mehreren Ultraschallwandlern 120 emittierte Ultraschallsignal das emittierte Sendesignal sein und das von den ein oder mehreren Sensoren 160 empfangene Signal das Messsignal (d.h. das empfangene Sensorsignal) sein.

Beispielsweise können die ein oder mehreren Sensoren ein oder mehrere optische Sensoren aufweisen (z.B. sein), wie beispielsweise Transmissionssensoren und/oder Reflexionssensoren. Die ein oder mehreren optischen Sensoren können eingerichtet sein, ein optisches Sensorsignal zu emittieren und anschließend ein optisches Messsignal zu empfangen. Beispielsweise können die ein oder mehreren optischen Sensoren eingerichtet sein, ein Licht mit einer bestimmten Wellenlänge zu emittieren. Beispielsweise kann das emittierte Licht an einer Oberfläche (z.B. einer jeweiligen Oberfläche von ein oder mehreren Strukturen innerhalb der Mundhöhle) reflektiert und/oder durch diese transmittiert werden. Im Anschluss kann das reflektierte bzw. transmittierte Licht durch einen der ein oder mehreren optischen Sensoren (z.B. einen anderen (bei Reflexion, Transmission) oder den gleichen (bei Reflexion)) empfangen werden. Das empfangene Lichtsignal kann von dem Sensor in ein Messsignal umgewandelt werden, das ein, mehrere oder alle der ein oder mehreren Eigenschaften der ein oder mehreren Strukturen innerhalb der Mundhöhle repräsentieren kann. Es versteht sich, dass in Kombination mit einer zuvor beschriebenen Beißschienen-Einlage 130, die Beißschienen-Einlage 130 an den zu den ein oder mehreren Sensoren 160 korrespondierenden Abschnitten für ein von den ein oder mehreren Sensoren 160 emittiertes und empfangenes Signal durchlässig ist.

Beispielsweise kann das mittels der ein oder mehreren Sensoren 160 empfangene Messsignal von den ein oder mehreren Sensoren 160 an die Steuervorrichtung 410 übermittelt werden, dargestellt durch den Pfeil 422. Die Steuervorrichtung kann eingerichtet sein, basierend auf dem Messsignal den einen, die mehreren oder alle der ein oder mehreren Eigenschaften der ein oder mehreren Strukturen innerhalb der Mundhöhle zu ermitteln. Ferner kann die Steuervorrichtung 410 eingerichtet sein, basierend auf dem Ermittlungsergebnis ein dazu korrespondierendes Ultraschallsignal oder eine Reihe von dazu korrespondierenden Ultraschallsignalen auszuwählen, die für eine Reinigung und/oder eine Regeneration am geeignetsten sind, und an die Beißschiene 110, insbesondere an die ein oder mehreren Ultraschallwandler 120 zu übermitteln.

Gemäß verschiedenen Aspekten kann die Steuervorrichtung 410 eingerichtet sein, einem Nutzer das Ermittlungsergebnis sowie das (oder mehrere) zum Ermittlungsergebnis korrespondierende Ultraschallsignal(e) bzw. die (oder mehrere) Reihe(n) von dazu korrespondierenden Ultraschallsignalen vorzuschlagen. Im Anschluss kann der Nutzer ein Ultraschallsignal oder eine Reihe von Ultraschallsignalen auswählen, welche dann von der Steuervorrichtung 410 an die Beißschiene 110, insbesondere an die Ultraschallwandler 120, übermittelt werden. Somit kann der Reinigungsprozess bzw. der Regenerationsprozess auf die Bedürfnisse des Benutzers abgestimmt werden.

Gemäß verschiedenen Aspekten kann ein Reinigungsvorgang und/oder ein Regenerationsvorgang aufgrund eines Koppelmediums verstärkt werden. Beispielsweise kann durch das Koppelmedium eine bessere Ultraschallwellenleitung zwischen den ein oder mehreren Ultraschallwandlern 120 und den Strukturen innerhalb des Mundraums gewährleistet werden (z.B. da keine Gasbläschen vorhanden sind). Beispielsweise kann das Koppelmedium ein oder mehrere Zusätze aufweisen, die einen Reinigungsprozess unterstützen können (z.B. Putzkörper) und/oder einen Regenerationsprozess unterstützen können (z.B. Fluorid, Hydroxylapatit, Zinkoxid (ZnO)).

Beispielsweise kann das Koppelmedium vor der Anwendung in die Beißschiene aufgetragen werden. Das birgt jedoch die Gefahr einer (lokalen) Überdosierung oder Unterdosierung durch einen Nutzer.

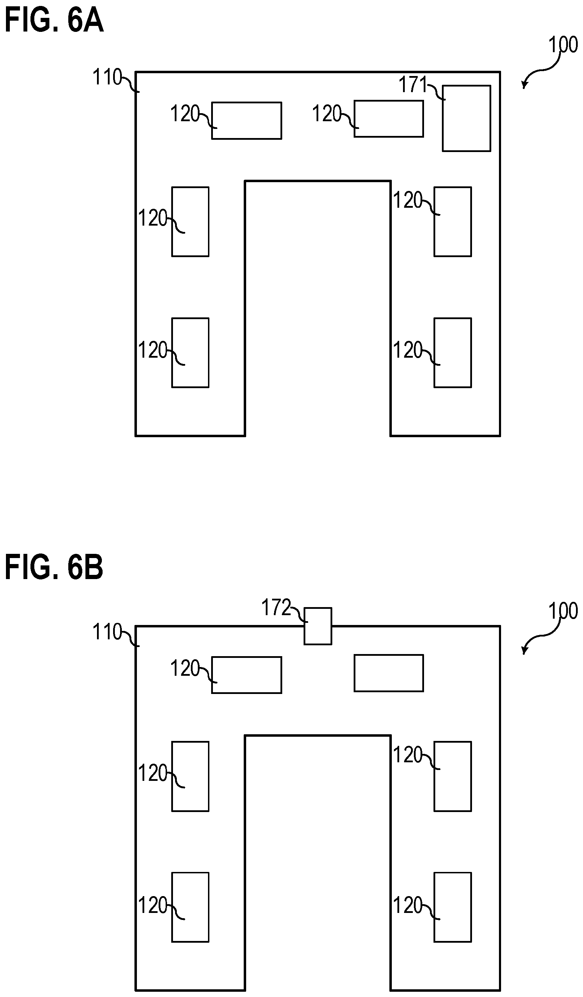

**Fig.6A** veranschaulicht schematisch ein Ultraschallsystem 100 gemäß verschiedenen Aspekten, das einen Koppelmedium-Speicher 171 zum Speichern eines Koppelmediums aufweist. Der Koppelmedium-Speicher 171 kann eingerichtet sein, eine bestimmte Menge des Koppelmediums abzugeben. Ferner kann der Koppelmedium-Speicher 171 eingerichtet sein, dem Nutzer zu signalisieren, wenn der Füllstand einen vorbestimmten Wert unterschreitet und/oder der Koppelmedium-Speicher 171 komplett leer ist.

Beispielsweise kann der Koppelmedium-Speicher 171 durch die Steuervorrichtung 410 gesteuert werden. Beispielsweise kann die Steuervorrichtung 410 eingerichtet sein, zusammen mit dem Ultraschallsignal, ein Dosierungssignal für den Koppelmedium-Speicher 171 an die Beißschiene 110 zu übermitteln. Das Dosierungssignal kann Informationen für den Koppelmedium-Speicher 171 aufweisen, wie viel des Koppelmediums über welchen Zeitraum abgegeben werden soll. Beispielsweise kann die Dosierung durch die Steuervorrichtung 410 auf die ermittelte(n) Eigenschaft(en) der ein oder mehreren Strukturen innerhalb der Mundhöhle angepasst sein. Ferner kann die Steuervorrichtung 410 eingerichtet sein, dem Nutzer zu signalisieren, wenn der Koppelmedium-Speicher 171 leer ist oder weniger als einen vorbestimmten Füllstand hat. Beispielsweise kann die Beißschiene 110 ein oder mehrere Koppelmedium-Verteilungsleitungen aufweisen, um das Koppelmedium gleichmäßig innerhalb der Beißschiene 110 zu verteilen. Somit kann eine kontinuierliche und individuell auf die Bedürfnisse eines Nutzers angepasste Koppelmedium-Versorgung gewährleistet werden.

**Fig.6B** veranschaulicht schematisch ein Ultraschallsystem 100 gemäß verschiedenen Aspekten, das einen Koppelmedium-Zuführelement 172 zum Zuführen eines Koppelmediums aufweist. Das Koppelmedium-Zuführelement 172 kann analog zum Koppelmedium-Speicher 171 ausgestaltet sein, wobei hierbei das Koppelmedium von außerhalb in die Beißschiene 110 zugeführt wird. Beispielsweise kann dazu ein separater Koppelmedium-Speicher, außerhalb der Beißschiene 110 bereitgestellt werden, der mit dem Koppelmedium-Zuführelement 172 verbunden ist.

Wie zuvor bereits beschrieben wurde, kann die Steuervorrichtung 410 eingerichtet sein, dem Benutzer verschiedene Zustände zu signalisieren. Dazu kann die Steuervorrichtung 410 ein oder mehrere Informationsanzeige-Elemente aufweisen.

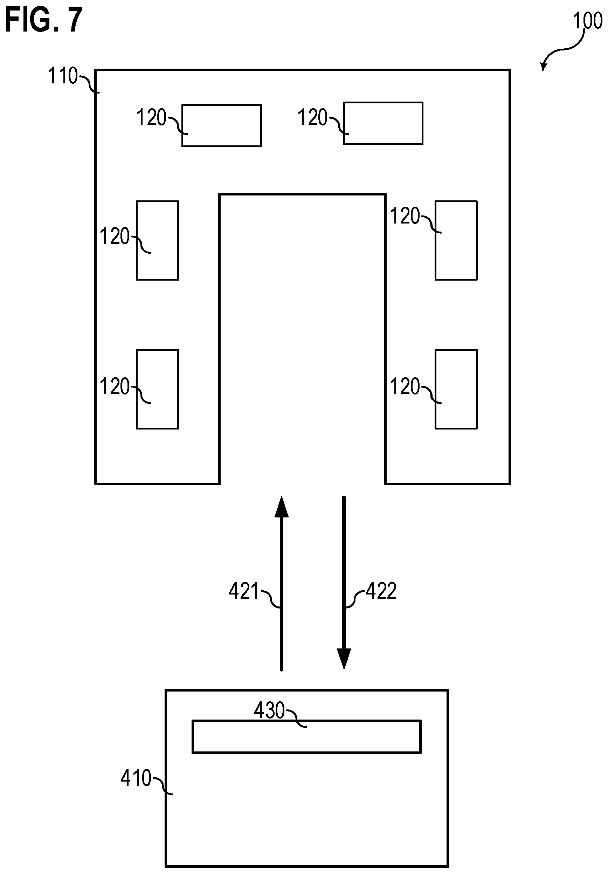

**Fig.7** veranschaulicht schematisch ein Ultraschallsystem 100 gemäß verschiedenen Aspekten, das ein Informationsanzeige-Element 430 zum Anzeigen einer Information aus ein oder mehreren Informationen aufweist.

Die ein oder mehreren Informationen können beispielsweise Ergebnisse der Ermittlung der ein oder mehreren Eigenschaften der ein oder mehreren Strukturen innerhalb der Mundhöhle aufweisen (z.B. sein). Die ein oder mehreren Informationen können beispielsweise Informationen über den Füllstand eines Energiespeichers des Ultraschallsystems 100 aufweisen (z.B. sein). Die ein oder mehreren Informationen können beispielsweise eine Information über einen Füllstand des Koppelmediums (z.B. im Koppelmedium-Speicher 171) aufweisen (z.B. sein). Die ein oder mehreren Informationen können beispielsweise eine Information über einen Fortschritt des Regenerationsprozesses und/oder Reinigungsprozesses aufweisen (z.B. sein). Beispielsweise können die ein oder mehreren Informationen eine Information darüber aufweisen, wann und/oder ob der Reinigungsprozess und/oder Regenerationsprozess abgeschlossen ist. Beispielsweise können die ein oder mehreren Informationen Fehlermeldungen aufweisen (z.B. sein).

Gemäß verschiedenen Aspekten können die ein oder mehreren Informationsanzeige-Elemente 430 ein oder mehrere optische Informationsanzeige-Elemente, akustische Informationsanzeige-Elemente und/oder haptische Informationsanzeige-Elemente aufweisen (z.B. sein).

Die ein oder mehreren optischen Informationsanzeige-Elemente können beispielsweise Leuchtelemente (z.B. Lampen, LED-Elemente etc.) sein. Beispielsweise können die ein oder mehreren Informationsanzeige-Elemente ein oder mehrere Bildschirme (z.B. Displays) aufweisen (z.B. sein), auf denen die Informationen angezeigt werden können.

Die ein oder mehreren akustischen Informationsanzeige-Elemente können beispielsweise Ton-Ausgabeelemente sein (z.B. Lautsprecher), die eingerichtet sind zum Ausgeben von Tönen. Somit kann beispielsweise eine akustische Fehlermeldung abgespielt werden, wenn ein Fehler auftritt. Beispielsweise kann über einen jeweiligen charakteristischen Ton der Beginn und/oder das Ende eines Reinigungsprozesses bzw. eines Regenerationsprozesses signalisiert werden.

Die ein oder mehreren haptischen Informationsanzeige-Elemente können beispielsweise Vibrations-Elemente sein (z.B. Vibrationsmotoren), die eingerichtet sind zum Vibrieren. Somit kann beispielsweise eine haptische Meldung durch eine charakteristische Vibrationsfolge bereitgestellt werden, wenn ein Fehler auftritt. Beispielsweise kann über eine jeweilige charakteristische Vibrationsfolge der Beginn und/oder das Ende eines Reinigungsprozesses bzw. eines Regenerationsprozesses signalisiert werden.

Gemäß verschiedenen Aspekten, kann die Beißschiene 110 auch ein oder mehrere haptische Informationsanzeige-Elemente aufweisen. Beispielsweise können die ein oder mehreren Ultraschallwandler 120 als haptische Informationsanzeige-Elemente eingerichtet sein. Somit kann beispielsweise ein charakteristisches Vibrieren in der Mundhöhle erzeugt werden, dass eine bestimmte Information repräsentiert.

Gemäß verschiedenen Aspekten kann das Ultraschallsystem 100 zwei Beißschienen 110 aufweisen. Jede der zwei Beißschienen 110 kann analog zu den Beschreibungen hierin ausgeführt sein/werden. Beispielsweise kann eine erste der zwei Beißschienen für den Oberkiefer eines Nutzers und die zweite der zwei Beißschienen für einen Unterkiefer des Nutzers angepasst sein. Die beiden Beißschienen können sowohl getrennt voneinander sein, als auch miteinander verbunden sein. Eine getrennte Ausführung kann beispielsweise einen erhöhten Tragekomfort gewährleisten. Eine Ausführung, in der die beiden Beißschienen verbunden sind, kann eventuell eine für einen dritten einfachere Anwendung bieten (z.B. in Verbindung mit einem Haltegriff), der insbesondere im Bereich der Pflege und/oder im Bereich der tierischen Zahnhygiene vorteilhaft sein kann. Ferner ermöglicht die verbundene Ausführung, dass weitere Bauteile (wie z.B. Energiespeicher etc.) innerhalb des Verbindungsteils angeordnet sein/werden können.

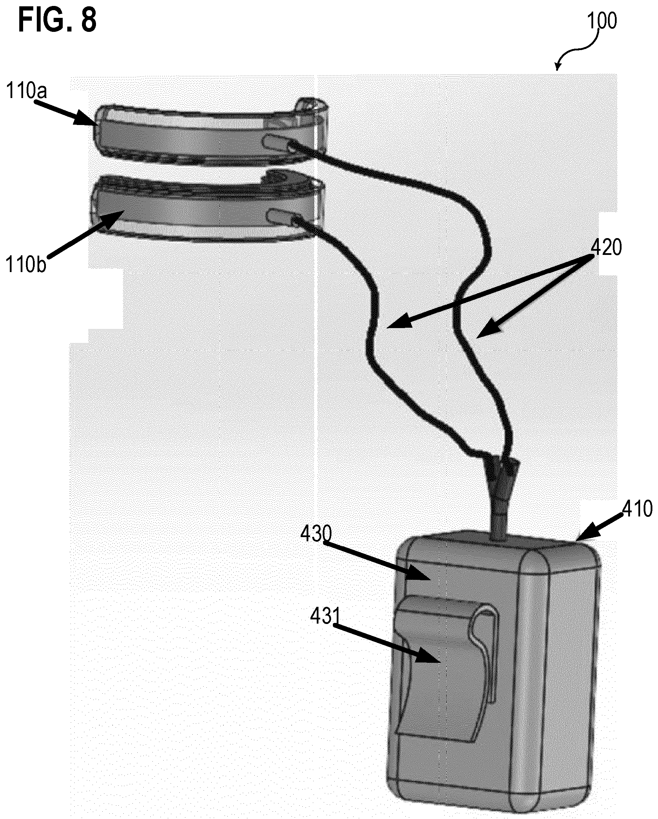

**Fig.8** veranschaulicht schematisch ein Ultraschallsystem 100 gemäß verschiedenen Aspekten, das zwei (separate) Beißschienen 110a, 110b aufweist. Beispielsweise kann die erste Beißschiene 110a für einen Oberkiefer eines Nutzers angepasst sein und die zweite Beißschiene 110b kann für den Unterkiefer des Nutzers angepasst sein. Ferner kann das Ultraschallsystem 100 eine Steuervorrichtung 410 aufweisen, die mit den zwei Beißschienen 110a, 110b über jeweils ein Verbindungselement 420 (z.B. ein Kabel, ein Seil) verbunden ist. Die Verbindungselemente 420 können für eine kabelgebundene Informationsübertragung zwischen der Steuervorrichtung und den jeweiligen Beißschienen 110a, 110b eingerichtet sein. Beispielsweise können die Verbindungselemente 420 auch lediglich Verbindungen sein, um die Beißschienen 110a, 110b nicht zu verlieren. In einem solchen Fall wäre die Informationsübertragung kabellos (wie zuvor beschrieben).

Beispielsweise kann die Steuervorrichtung 410 notwendige Elektronikkomponenten zur Energieversorgung, Steuerung, Messdatenaufzeichnung, und/oder Messdatenauswertung aufweisen.

Beispielsweise kann die Steuervorrichtung 410 ein Gehäuse 431 aufweisen. Das Gehäuse 431 zeichnet sich durch seine geringen geometrischen Dimensionen, ein niedriges bis moderates Gewicht (z.B. vergleichbar mit einem Smartphone, beispielsweise weniger als 500 g oder beispielsweise weniger als 350 g oder beispielsweise weniger als 200 g) und/oder der Möglichkeit der Befestigung an Bekleidungsstücken des Nutzers durch ein Befestigungselement 431 aus. Es versteht sich, dass eine leichtere Steuervorrichtung 410 einfacher an einem Kleidungsstück eines Nutzers angebracht werden kann.

Beispielsweise kann die Beißschiene 110 in verschiedenen Universalgrößen realisiert sein/werden, die jeweils für einen groben Nutzerkreis anwendbar sind, z.B. für Erwachsene, Kinder, Männer, Frauen, Katzen, Hunde, Pferde oder sonstige Tiere. Die Beißschiene 110 kann mehrere Ultraschallwandler 120, z.B. Ultraschallemitter bzw. (Ultraschall-)Aktoren, und/oder ein oder mehrere Sensoren 160 aufweisen, die jeweils gemäß den Beschreibungen hierin ausgestaltet sein können. Beispielsweise können die mehreren Ultraschallwandler 120 fest in der Beißschiene 110 (z.B. in einer eine Beißschienen-Hülle 140) verbaut sein. Beispielsweise können die ein oder mehreren Sensoren 160 fest in der Beißschiene 110 (z.B. in einer Beißschienen-Hülle 140) verbaut sein.

Beispielsweise kann, für eine individuelle Anpassung des Ultraschallsystems 100 an eine Gebissform eines Nutzers eine Beißschienen-Einlage 130 verwendet werden. Die Beißschienen-Einlage 130 kann eine oder mehrere Polymere und/oder ein oder mehrere thermoplastische Werkstoffe aufweisen. Beispielsweise kann die Beißschienen-Einlage 130 Silikon und/oder Ethylenvinylacetat (EVA) und/oder Polymethylmethacrylat (PMMA) aufweisen (z.B. daraus bestehen). Die Beißschienen-Einlage 130 kann von einem Nutzer auf dessen Gebissform angepasst werden. Beispielsweise kann die Beißschienen-Einlage 130 eingerichtet sein, verformbar zu sein, wenn sie über eine bestimmte Temperatur erwärmt wird, und nach einem Erkalten diese Form beizubehalten (d.h. im Wesentlichen aus einem thermoplastischen Werkstoff besteht). In diesem Fall kann der Nutzer die Beißschienen-Einlage 130 erwärmen (z.B. in einem Wasserbad) und anschließend die Beißschienen-Einlage 130 an seine Zahnform anpassen. Es versteht sich, dass auch extern angepasste (z.B. durch einen Zahnarzt, z.B. analog zu herkömmlichen Gebissabdrücken) Beißschienen-Einlagen 130 verwendet werden können.

Beispielsweise kann die Beißschienen-Einlage 130 vor der Anpassung ein Abstandshalter-Element aufweisen, beispielsweise in Form einer ablösbaren Schicht (z.B. einer Folie). Nach der Anpassung an die Gebissform des Nutzers kann das Abstandshalter-Element von der Beißschienen-Einlage 130 entfernt werden. Somit kann ein Zwischenraum zwischen der Beißschienen-Einlage 130 und dem Gebiss des Nutzers entstehen. Alternativ dazu, kann die Beißschienen-Einlage 130 auch derart ausgestaltet sein, dass sie beim Erkalten einen Zwischenraum zu den Zähnen bildet. Beispielsweise kann die Beißschienen-Einlage 130 auch von vornerein einen Zwischenraum aufweisen, der durch die individuelle Anpassung nicht oder nur unwesentlich (d.h. ohne Funktionseinbußen) verändert wird. Der Zwischenraum ermöglicht beispielsweise, dass ein Koppelmedium (z.B. eine Zahnpasta) eingebracht werden kann. Somit können günstige Effekte durch eine Kombination des Koppelmedium und der Ultraschallwellen erreicht werde. Beispielsweise können die mehreren Ultraschallwandler 120 Ultraschallwellen erzeugen wodurch physikalischen Effekte (Kavitation, mechanische Reibung der Putzkörper des Koppelmediums, Remineralisierung etc.) und/oder biologischen Effekte (z.B. eine Anregung zur Zellproliferation, eine Anregung zur Bildung von Proteinen) an und/oder in den ein oder mehreren Strukturen innerhalb der Mundhöhle erzeugt werden, die zu einer Reinigung und/oder Regeneration der ein oder mehreren Strukturen innerhalb der Mundhöhle führen können.

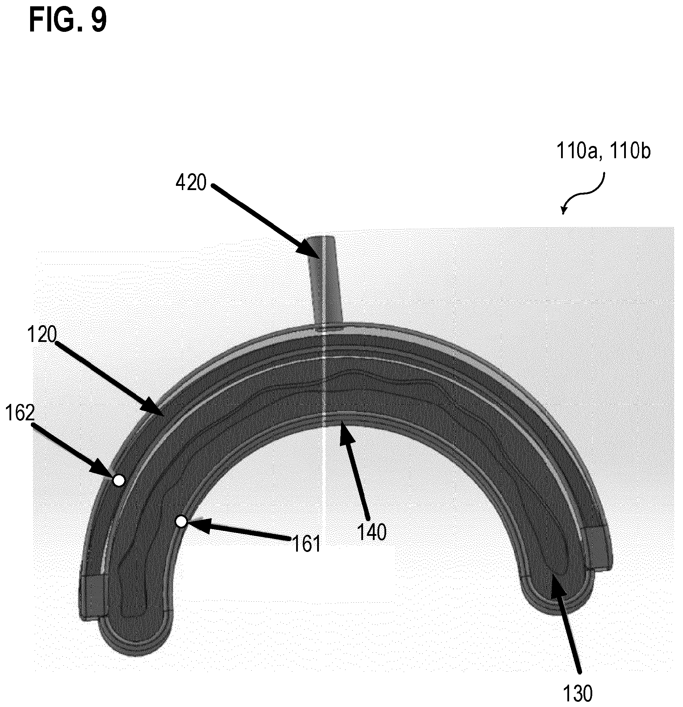

**Fig.9** veranschaulicht beispielhaft eine Beißschiene 110 der zwei Beißschienen 110a, 110b aus Fig.8 in einer Ansicht, in der die Beißschienen-Einlage 130 sichtbar ist. Wie dargestellt, kann die Beißschienen-Einlage 130 in die Beißschienenhülle eingesetzt sein. Gemäß verschiedenen Aspekten kann die Beißschiene 110 ein oder mehrere Ultraschallwandler 120 aufweisen. Die ein oder mehreren Ultraschallwandler 120 können in einem Modul zusammengefasst sein. Dieses erlaubt beispielsweise einen schnellen Austausch, wenn einer der ein oder mehreren Ultraschallwandler 120 defekt ist. Ferner kann somit beispielsweise ein homogenes Ultraschallwellenfeld erzeugt werden.

Gemäß verschiedenen Aspekten können in der Beißschiene 110 ein oder mehrere Sensoren 160 angeordnet sein. Beispielsweise können ein oder mehrere der ein oder mehreren Sensoren 160 (z.B. alle) jeweils ein erstes Sensorelement 161 und ein zweites Sensorelement 162 aufweisen. Beispielsweise kann das erste Sensorelement 161 ein Sensorsignal-Bereitstellungselement zum Bereitstellen des Sensorsignals sein und das zweite Sensorelement 162 kann ein Messsignal-Empfangselement zum Empfangen des Messsignals sein. Wie zuvor beschrieben kann das erste Sensorelement 161 ein optisches Sensorelement sein, das eingerichtet ist, Licht (z.B. ein UV-Licht, Infrarot-Licht, sichtbares Licht), das ein oder mehrere vorbestimmte Wellenlängen aufweist, zu emittieren. Das Licht kann von den ein oder mehreren Strukturen innerhalb der Mundhöhle transmittiert und/oder reflektiert werden und als Messsignal von einem (oder mehreren) zweiten Sensorelementen 162 empfangen werden.

Es versteht sich, dass, obwohl in Fig.9 nur beispielhaft ein erstes Sensorelement 161 und ein zweites Sensorelement 162 dargestellt sind, die Beißschiene 110 eine Mehrzahl von ersten Sensorelementen 161 und eine Mehrzahl von zweiten Sensorelementen 162 aufweisen kann.

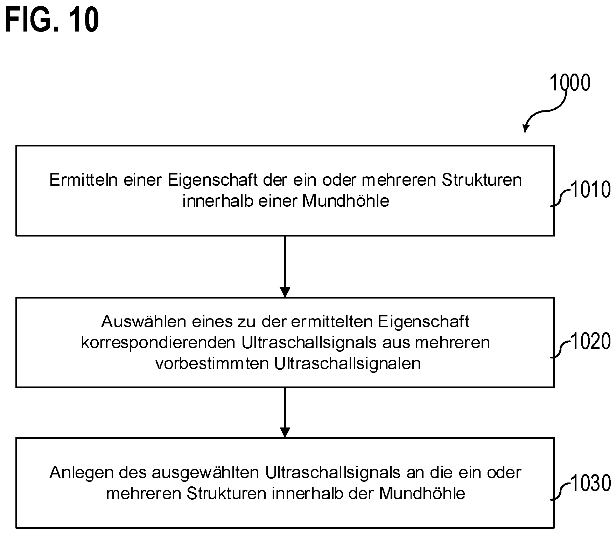

**Fig.10** veranschaulicht beispielhaft ein Verfahren zum Reinigen und/oder Regenerieren von ein oder mehreren Strukturen innerhalb der Mundhöhle. Es versteht sich, dass ein derartiges Verfahren beispielsweise mit einem hierin beschriebenen Ultraschallsystem 100 durchgeführt werden kann. Das Verfahren kann aufweisen: Ermitteln einer Eigenschaft der ein oder mehreren Strukturen innerhalb der Mundhöhle 1010. Das Ermitteln kann beispielsweise mittels der ein oder mehreren Sensoren 160 und der Steuervorrichtung 410 wie zuvor beschrieben durchgeführt werden.

Ferner kann das Verfahren aufweisen, Auswählen eines zu der ermittelten Eigenschaft korrespondierenden Ultraschallsignals aus mehreren vorbestimmten Ultraschallsignalen 1020. Diese Auswahl kann beispielsweise durch die Steuervorrichtung 410 durchgeführt werden.

Ferner kann das Verfahren aufweisen, Anlegen des ausgewählten Ultraschallsignals an die ein oder mehreren Strukturen innerhalb der Mundhöhle 1030. Beispielsweise kann die Steuervorrichtung 410 ein geeignetes Ultraschallsignal, basierend auf der zuvor ermittelten Eigenschaft auswählen und dieses an die mehreren Ultraschallwandler 120 innerhalb der Beißschiene übermitteln.

Gemäß verschiedenen Aspekten wird ein Ultraschallsystem bereitgestellt, das eine nichtabrasive Reinigung von Zähnen, Zahnoberflächen, Interdentalräumen, eines Zahnhalteapparats und von an den Zähnen (direkt und/oder indirekt) angrenzenden Strukturen ermöglicht. Das Ultraschallsystem kann beispielsweise einen Reinigungseffekt, der nicht von einer manuellen Führung des Zahnreinigungsgeräts abhängig ist, ermöglichen, wie beispielsweise bei einer herkömmlichen Zahnbürste. Das Ultraschallsystem kann ein gleichmäßigeres Reinigungsergebnis als herkömmliche Zahnbürsten ermöglichen.

Gemäß verschiedenen Aspekten wird ein Ultraschallsystem zur Zahnreinigung bereitgestellt, nach dessen Verwendung auf eine zusätzliche Zahnzwischenraumpflege verzichtet werden kann.

Gemäß verschiedenen Aspekten wird ein Ultraschallsystem zur Zahnreinigung bereitgestellt, das eine vereinfachte Zahnreinigung für Personen ermöglichen kann, die aufgrund ihres Alters oder ihrer Gesundheit beeinträchtigt sind.

Gemäß verschiedenen Aspekten wird ein Ultraschallsystem zur Zahnreinigung bereitgestellt, das eine Regenerationsstimulation der Zahnhartsubstanz, des Zahnhalteapparats und des Kieferknochens stimulieren kann.

Gemäß verschiedenen Aspekten wird ein Ultraschallsystem zur Zahnreinigung bereitgestellt, das geräuscharm angewendet werden kann.

Im Folgenden werden einige Beispiele beschrieben, die sich auf das hierin Beschriebene und in den Figuren Dargestellte beziehen.

## Patentansprüche

1. Ultraschallsystem (100) aufweisend:
eine Steuervorrichtung (410), die eingerichtet ist zum Auswählen und Bereitstellen eines ausgewählten Ultraschallsignals, das aus mehreren auswählbaren Ultraschallsignalen auswählbar ist, die sich in ihren jeweiligen Frequenzbereichen, Intensitäten und/oder Pulsfrequenzen unterscheiden;
eine Beißschiene (110) zum Einbringen und Positionieren in einer Mundhöhle,
wobei die Beißschiene (110) ein oder mehrere Ultraschallwandler (120) zum Anlegen eines ausgewählten Ultraschallsignals an ein oder mehrere Strukturen innerhalb einer Mundhöhle zum Reinigen und/oder zur Regeneration der ein oder mehreren Strukturen innerhalb der Mundhöhle aufweist;
wobei die Beißschiene (110) ferner ein oder mehrere optische Sensoren (160) aufweist zum Ermitteln einer Eigenschaft der ein oder mehreren Strukturen innerhalb der Mundhöhle; und
wobei die Steuervorrichtung (410) eingerichtet ist, das ausgewählte Ultraschallsignal basierend auf der ermittelten Eigenschaft der ein oder mehreren Strukturen innerhalb der Mundhöhle auszuwählen und bereitzustellen.

2. Ultraschallsystem (100) gemäß Anspruch 1, wobei die Beißschiene (110) aufweist:
eine Beißschienen-Hülle (140), in der die ein oder mehreren Ultraschallwandler (120) positioniert sind, und
eine Beißschienen-Einlage (130), die an die ein oder mehreren Strukturen innerhalb der Mundhöhle anpassbar ist, und die eingerichtet ist, reversibel in der Beißschienen-Hülle (140) befestigt zu werden.

3. Ultraschallsystem (100) gemäß Anspruch 2, wobei
die Beißschienen-Einlage (130) ein thermoplastisches Material aufweist, derart dass es aufgrund einer Erwärmung an die ein oder mehreren Strukturen innerhalb der Mundhöhle angepasst werden kann.

4. Ultraschallsystem (100) gemäß Anspruch 2 oder 3, wobei die Beißschiene (110) ferner aufweist:
ein oder mehrere Befestigungselemente (150) zum reversiblen Befestigen der Beißschienen-Einlage in der Beißschienen-Hülle,
wobei die ein oder mehreren Befestigungselemente (150) vorzugsweise ein oder mehrere der folgenden Elemente aufweisen: mechanische Befestigungselemente zum mechanischen Befestigen der Beißschienen-Einlage (130) in der Beißschienen-Hülle (140), magnetische Elemente zum magnetischen Befestigen der Beißschienen-Einlage (130) in der Beißschienen-Hülle (140), und/oder haftende Elemente zum Anhaften der Beißschienen-Einlage (130) in der Beißschienen-Hülle (140).

5. Ultraschallsystem (100) gemäß einem der Ansprüche 1 bis **4,**
wobei die ein oder mehreren Ultraschallwandler (120) ein oder mehrere Ultraschallemitter aufweisen.

6. Ultraschallsystem (100) gemäß Anspruch 1,
wobei ein auswählbares Ultraschallsignal der ein oder mehreren auswählbaren Ultraschallsignale dazu geeignet ist, eine Regeneration zu stimulieren, und
wobei vorzugsweise ein auswählbares Ultraschallsignal zum Stimulieren einer Regeneration eingerichtet ist zum Erzeugen von Ultraschallwellen in einem HIFU-Bereich und/oder einem LIPUS-Bereich.

7. Ultraschallsystem (100) gemäß Anspruch 1 oder 6,
wobei ein auswählbares Ultraschallsignal der ein oder mehreren auswählbaren Ultraschallsignale dazu geeignet ist, Verunreinigungen von ein oder mehreren Oberflächen der ein oder mehreren Strukturen innerhalb der Mundhöhle zu entfernen, und
wobei vorzugsweise ein auswählbares Ultraschallsignal zum Entfernen von Verunreinigungen eine Frequenz im Bereich zwischen 1 kHz und 20 MHz, eine Intensität zwischen 0,01 W/cm² und 1 kW/cm² aufweist, und eine Pulsfrequenz zwischen 10 Hz und 20 kHz aufweist.

8. Ultraschallsystem (100) gemäß Anspruch 1 oder gemäß einem der Ansprüche 6 bis 7,
wobei ein auswählbares Ultraschallsignal der ein oder mehreren auswählbaren Ultraschallsignale dazu geeignet ist, Zahnstein von ein oder mehreren Oberflächen der ein oder mehreren Strukturen innerhalb der Mundhöhle zu entfernen, und
wobei vorzugsweise ein auswählbares Ultraschallsignal zum Entfernen von Zahnstein eine Frequenz im Bereich zwischen 1 kHz und 20 MHz aufweist, eine Intensität zwischen 0,01 W/cm² und 10 kW/cm², aufweist, und eine Pulsfrequenz zwischen 10 Hz und 20 kHz aufweist.

9. Ultraschallsystem (100) gemäß einem der Ansprüche 6 bis 8,
wobei die Beißschiene (110) ein oder mehrere Sensoren (160), vorzugsweise ein oder mehrere optische Sensoren, aufweist zum Ermitteln einer Eigenschaft der ein oder mehreren Strukturen innerhalb der Mundhöhle; und
wobei das ausgewählte Ultraschallsignal der ermittelten Eigenschaft der ein oder mehreren Strukturen innerhalb der Mundhöhle zugeordnet ist.

10. Ultraschallsystem (100) gemäß Anspruch 1 oder 9,
wobei die Steuervorrichtung (410) ferner eingerichtet ist zum Ermitteln der Eigenschaft der ein oder mehreren Strukturen innerhalb der Mundhöhle mittels der ein oder mehreren Sensoren (160), und
wobei die Steuervorrichtung (410) ferner eingerichtet ist, das Ultraschallsignal basierend auf der ermittelten Eigenschaft auszuwählen und bereitzustellen.

11. Ultraschallsystem (100) gemäß Anspruch 10,
wobei die Steuervorrichtung (410) ferner eingerichtet ist zum Erhöhen einer Intensität und/oder zum Verringern der Intensität des ausgewählten Ultraschallsignals in Abhängigkeit von einer Ausprägung der ermittelten Eigenschaft der ein oder mehreren Strukturen innerhalb der Mundhöhle; und
wobei die Steuervorrichtung (410) vorzugsweise ferner eingerichtet ist zum Starten und/oder Stoppen des ausgewählten Ultraschallsignals in Abhängigkeit von einer Anwesenheit bzw. einer Abwesenheit der ermittelten Eigenschaft der ein oder mehreren Strukturen innerhalb der Mundhöhle.

12. Ultraschallsystem (100) gemäß einem der Ansprüche 1 bis 11,
wobei das Ultraschallsystem (100), vorzugsweise die Beißschiene (110), borstenfrei ist.

## Claims

1. Ultrasound system (100) having:
a control device (410) which is designed for selecting and providing a selected ultrasound signal which can be selected from amongst a plurality of selectable ultrasound signals which differ in their respective frequency ranges, intensities and/or pulse frequencies;
a bite tray (110) for insertion and positioning in an oral cavity,
wherein the bite tray (110) has one or more ultrasound transducers (120) for applying a selected ultrasound signal to one or more structures within an oral cavity for cleaning and/or for regenerating the one or more structures within the oral cavity;
wherein the bite tray (110) further has one or more optical sensors (160) for determining a property of the one or more structures within the oral cavity; and
wherein the control device (410) is designed to select and provide the selected ultrasound signal based on the determined property of the one or more structures within the oral cavity.

2. Ultrasound system (100) according to Claim 1, wherein the bite tray (110) has:
a bite tray sleeve (140) in which the one or more ultrasound transducers (120) are positioned, and
a bite tray insert (130) which can be adapted to the one or more structures within the oral cavity and which is designed to be reversibly fastened in the bite tray sleeve (140).

3. Ultrasound system (100) according to Claim 2, wherein
the bite tray insert (130) contains a thermoplastic material such that it can be adapted to the one or more structures within the oral cavity on account of being heating.

4. Ultrasound system (100) according to Claim 2 or 3, wherein the bite tray (110) further has:
one or more fastening elements (150) for reversibly fastening the bite tray insert in the bite tray sleeve, wherein the one or more fastening elements (150) preferably have one or more of the following elements: mechanical fastening elements for mechanically fastening the bite tray insert (130) in the bite tray sleeve (140), magnetic elements for magnetically fastening the bite tray insert (130) in the bite tray sleeve (140), and/or adhesive elements for adhesively bonding the bite tray insert (130) in the bite tray sleeve (140).

5. Ultrasound system (100) according to any of Claims 1 to 4,
wherein the one or more ultrasound transducers (120) have one or more ultrasound emitters.

6. Ultrasound system (100) according to Claim 1,
wherein a selectable ultrasound signal of the one or more selectable ultrasound signals is suitable for stimulating regeneration, and
wherein preferably a selectable ultrasound signal for stimulating regeneration is designed for generating ultrasonic waves in an HIFU range and/or a LIPUS range.

7. Ultrasound system (100) according to Claim 1 or 6,
wherein a selectable ultrasound signal of the one or more selectable ultrasound signals is suitable for removing impurities from one or more surfaces of the one or more structures within the oral cavity, and
wherein preferably a selectable ultrasound signal for removing impurities has a frequency in the range between 1 kHz and 20 MHz, an intensity between 0.01 W/cm² and 1 kW/cm², and a pulse frequency between 10 Hz and 20 kHz.

8. Ultrasound system (100) according to Claim 1 or according to either of Claims 6 and 7,
wherein a selectable ultrasound signal of the one or more selectable ultrasound signals is suitable for removing tartar from one or more surfaces of the one or more structures within the oral cavity, and
wherein preferably a selectable ultrasound signal for removing tartar has a frequency in the range between 1 kHz and 20 MHz, an intensity between 0.01 W/cm² and 10 kW/cm², and a pulse frequency between 10 Hz and 20 kHz.

9. Ultrasound system (100) according to any of Claims 6 to 8,
wherein the bite tray (110) has one or more sensors (160), preferably one or more optical sensors, for determining a property of the one or more structures within the oral cavity; and
wherein the selected ultrasound signal is assigned to the determined property of the one or more structures within the oral cavity.

10. Ultrasound system (100) according to Claim 1 or 9,
wherein the control device (410) is further designed for determining the property of the one or more structures within the oral cavity by means of the one or more sensors (160), and
wherein the control device (410) is further designed to select and provide the ultrasound signal based on the determined property.

11. Ultrasound system (100) according to Claim 10,
wherein the control device (410) is further designed for increasing an intensity and/or for reducing the intensity of the selected ultrasound signal as a function of a characteristic of the determined property of the one or more structures within the oral cavity; and
wherein the control device (410) is preferably further designed for starting and/or stopping the selected ultrasound signal as a function of the presence or absence of the determined property of the one or more structures within the oral cavity.

12. Ultrasound system (100) according to any of Claims 1 to 11,
wherein the ultrasound system (100), preferably the bite tray (110), is free of bristles.

## Revendications

1. Système à ultrasons (100) présentant :
un dispositif de commande (410) qui est configuré pour sélectionner et fournir un signal ultrasonore sélectionné, pouvant être sélectionné parmi plusieurs signaux ultrasonores sélectionnables qui se distinguent par leurs plages de fréquences respectives, leurs intensités et/ou leurs fréquences d'impulsion respectives ;
une gouttière de morsure (110) destinée à être introduite et positionnée dans une cavité buccale,
dans lequel la gouttière de morsure (110) présente un ou plusieurs transducteurs à ultrasons (120) destinés à appliquer un signal ultrasonore sélectionné à une ou plusieurs structures à l'intérieur d'une cavité buccale afin de nettoyer et/ou de régénérer les une ou plusieurs structures à l'intérieur de la cavité buccale ;
dans lequel la gouttière de morsure (110) présente en outre un ou plusieurs capteurs optiques (160) destinés à déterminer une propriété des une ou plusieurs structures à l'intérieur de la cavité buccale ; et
dans lequel le dispositif de commande (410) est configuré pour sélectionner et fournir le signal ultrasonore sélectionné sur la base de la propriété déterminée des une ou plusieurs structures à l'intérieur de la cavité buccale.

2. Système à ultrasons (100) selon la revendication 1, dans lequel la gouttière de morsure (110) présente :
une enveloppe de gouttière de morsure (140), dans laquelle les un ou plusieurs transducteurs à ultrasons (120) sont positionnés, et
une garniture de gouttière de morsure (130), qui est adaptable aux une ou plusieurs structures à l'intérieur de la cavité buccale et qui est configurée pour être fixée de manière réversible dans l'enveloppe de gouttière de morsure (140).

3. Système à ultrasons (100) selon la revendication 2, dans lequel
la garniture de gouttière de morsure (130) présente un matériau thermoplastique, de telle sorte qu'elle puisse être adaptée aux une ou plusieurs structures à l'intérieur de la cavité buccale sous l'effet d'un chauffage.

4. Système à ultrasons (100) selon la revendication 2 ou 3, dans lequel la gouttière de morsure (110) présente en outre :
un ou plusieurs éléments de fixation (150) destinés à fixer de manière réversible la garniture de gouttière de morsure dans l'enveloppe de gouttière de morsure,
dans lequel les un ou plusieurs éléments de fixation (150) présentent de préférence un ou plusieurs des éléments suivants : des éléments de fixation mécaniques destinés à fixer mécaniquement la garniture de gouttière de morsure (130) dans l'enveloppe de gouttière de morsure (140), des éléments magnétiques destinés à fixer magnétiquement la garniture de gouttière de morsure (130) dans l'enveloppe de gouttière de morsure (140), et/ou des éléments adhésifs destinés à faire adhérer la garniture de gouttière de morsure (130) dans l'enveloppe de gouttière de morsure (140).

5. Système à ultrasons (100) selon l'une quelconque des revendications 1 à 4, dans lequel
les un ou plusieurs transducteurs à ultrasons (120) présentent un ou plusieurs émetteurs à ultrasons.

6. Système à ultrasons (100) selon la revendication 1, dans lequel
un signal ultrasonore sélectionnable parmi les un ou plusieurs signaux ultrasonores sélectionnables est apte à stimuler une régénération, et
et dans lequel, de préférence, un signal ultrasonore sélectionnable destiné à stimuler une régénération est configuré pour générer des ondes ultrasonores dans une plage HIFU et/ou une plage LIPUS.

7. Système à ultrasons (100) selon la revendication 1 ou 6, dans lequel
un signal ultrasonore sélectionnable parmi les un ou plusieurs signaux ultrasonores sélectionnables est apte à éliminer des impuretés d'une ou de plusieurs surfaces des une ou plusieurs structures à l'intérieur de la cavité buccale, et
et dans lequel, de préférence, un signal ultrasonore sélectionnable destiné à éliminer des impuretés présente une fréquence comprise entre 1 kHz et 20 MHz, une intensité comprise entre 0,01 W/cm² et 1 kW/cm², et une fréquence d'impulsion comprise entre 10 Hz et 20 kHz.

8. Système à ultrasons (100) selon la revendication 1 ou selon l'une quelconque des revendications 6 à 7, dans lequel
un signal ultrasonore sélectionnable parmi les un ou plusieurs signaux ultrasonores sélectionnables est apte à éliminer du tartre d'une ou de plusieurs surfaces des une ou plusieurs structures à l'intérieur de la cavité buccale, et
et dans lequel, de préférence, un signal ultrasonore sélectionnable destiné à éliminer le tartre présente une fréquence comprise entre 1 kHz et 20 MHz, une intensité comprise entre 0,01 W/cm² et 10 kW/cm², et une fréquence d'impulsion comprise entre 10 Hz et 20 kHz.

9. Système à ultrasons (100) selon l'une quelconque des revendications 6 à 8, dans lequel
dans lequel la gouttière de morsure (110) présente un ou plusieurs capteurs (160), de préférence un ou plusieurs capteurs optiques, destinés à déterminer une propriété des une ou plusieurs structures à l'intérieur de la cavité buccale ; et
et dans lequel le signal ultrasonore sélectionné est associé à la propriété déterminée des une ou plusieurs structures à l'intérieur de la cavité buccale.

10. Système à ultrasons (100) selon la revendication 1 ou 9, dans lequel
dans lequel le dispositif de commande (410) est en outre configuré pour déterminer la propriété des une ou plusieurs structures à l'intérieur de la cavité buccale au moyen des un ou plusieurs capteurs (160), et
dans lequel le dispositif de commande (410) est en outre configuré pour sélectionner et fournir le signal ultrasonore sur la base de la propriété déterminée.

11. Système à ultrasons (100) selon la revendication 10, dans lequel
dans lequel le dispositif de commande (410) est en outre configuré pour augmenter une intensité et/ou diminuer l'intensité du signal ultrasonore sélectionné en fonction d'un degré de manifestation de la propriété déterminée des une ou plusieurs structures à l'intérieur de la cavité buccale ; et
dans lequel le dispositif de commande (410) est de préférence en outre configuré pour démarrer et/ou arrêter le signal ultrasonore sélectionné en fonction de la présence ou de l'absence de la propriété déterminée des une ou plusieurs structures à l'intérieur de la cavité buccale.

12. Système à ultrasons (100) selon l'une quelconque des revendications 1 à 11, dans lequel
dans lequel le système à ultrasons (100), de préférence la gouttière de morsure (110), est dépourvu de poils.
